(19) Europäisches Patentamt / European Patent Office / Office européen des brevets

(11) **EP 2 912 198 B1**

(12) **EUROPEAN PATENT SPECIFICATION**

(45) Date of publication and mention
of the grant of the patent:
**22.11.2017 Bulletin 2017/47**

(21) Application number: **13792604.4**

(22) Date of filing: **24.10.2013**

(51) Int Cl.:
*C12R 1/01* (2006.01)    *A61K 39/02* (2006.01)
*C12N 1/20* (2006.01)    *C12N 1/36* (2006.01)

(86) International application number:
**PCT/EP2013/072319**

(87) International publication number:
**WO 2014/064217 (01.05.2014 Gazette 2014/18)**

(54) **IMMUNOGENIC COMPOSITION AGAINST AEROMONAS HYDROPHILA**

IMMUNOGENE ZUSAMMENSETZUNG GEGEN AEROMONAS HYDROPHILA

COMPOSITION IMMUNOGÈNE CONTRE AEROMONAS HYDROPHILA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.10.2012 VN 201203153
18.06.2013 DK 201370328**

(43) Date of publication of application:
**02.09.2015 Bulletin 2015/36**

(73) Proprietor: **Pharmaq AS
7863 Overhalla (NO)**

(72) Inventors:
• **TUNG, Vo Thanh**
**Ho Chi Minh Ci (VN)**
• **PHUONG, Tran Duy**
**Tien Giang pro (VN)**
• **PHUONG, Le Thi Kim**
**Da Lat City (VN)**
• **PHUC, Nguyen Truong**
**An Giang Province**
**An Giang Provi (VN)**
• **NYGAARD, Anja**
**1165 Oslo (NO)**
• **HUNGERHOLDT, Liv Blom**
**3512 Hønefoss (NO)**
• **KLEVAN, Leif Are**
**0661 Oslo (NO)**

(74) Representative: **Mannion, Sally Kim**
**Zoetis UK Limited**
**Walton Oaks**
**Dorking Road**
**Tadworth**
**Surrey KT20 7NS (GB)**

(56) References cited:
**WO-A2-2010/099444      CN-A- 101 642 567
US-A1- 2012 258 139**

• **M. E. MARTINO ET AL: "Determination of
Microbial Diversity of Aeromonas Strains on the
Basis of Multilocus Sequence Typing,
Phenotype, and Presence of Putative Virulence
Genes", APPLIED AND ENVIRONMENTAL
MICROBIOLOGY, vol. 77, no. 14, 3 June 2011
(2011-06-03), pages 4986-5000, XP055099971,
ISSN: 0099-2240, DOI: 10.1128/AEM.00708-11
cited in the application -& DATABASE EMBL
[Online] 25 July 2011 (2011-07-25), "Aeromonas
sp. Ae62(2011) strain Ae62 type II citrate synthase
(gltA) gene, partial cds.", XP002720189, retrieved
from EBI accession no. EM_STD:JF323311
Database accession no. JF323311 -& DATABASE
EMBL [Online] 25 July 2011 (2011-07-25),
"Aeromonas sp. Ae61(2011) strain Ae61
methionyl-tRNA synthetase (metG) gene, partial
cds.", XP002720190, retrieved from EBI
accession no. EM_STD:JF323393 Database
accession no. JF323393**

- M CRUMLISH ET AL: "Experimental challenge studies in Vietnamese catfish, Pangasianodon hypophthalmus (Sauvage), exposed to Edwardsiella ictaluri and Aeromonas hydrophila", JOURNAL OF FISH DISEASES, vol. 33, no. 9, 21 September 2010 (2010-09-21), pages 717-722, XP055099933, ISSN: 0140-7775, DOI: 10.1111/j.1365-2761.2010.01173.x
- JULIA W PRIDGEON ET AL: "Development and efficacy of novobiocin and rifampicin-resistant as novel vaccines in channel catfish and Nile tilapia", VACCINE, ELSEVIER LTD, GB, vol. 29, no. 45, 15 August 2011 (2011-08-15), pages 7896-7904, XP028311161, ISSN: 0264-410X, DOI: 10.1016/J.VACCINE.2011.08.082 [retrieved on 2011-08-18]
- M.R. CHANDRAN ET AL: "Immunisation of Indian major carps against Aeromonas hydrophila by intraperitoneal injection", FISH & SHELLFISH IMMUNOLOGY, vol. 13, no. 1, 1 July 2002 (2002-07-01), pages 1-9, XP055100291, ISSN: 1050-4648, DOI: 10.1006/fsim.2001.0374
- J. M. JANDA ET AL: "The Genus Aeromonas: Taxonomy, Pathogenicity, and Infection", CLINICAL MICROBIOLOGY REVIEWS, vol. 23, no. 1, 1 January 2010 (2010-01-01), pages 35-73, XP055100486, ISSN: 0893-8512, DOI: 10.1128/CMR.00039-09

SEQUENCE LISTING

<110> PHARMAQ AS
Tung, Vo Thanh
Phuong, Tran Dyu
Phouong, Li Thi Kim
Nguyen, Phuc
Nygaard, Anja
Hungerholdt, Liv Blom
Klevan, Leif Are

<120> Immunogenic composition against Aeromonas hydrophila

<130> 49902dk02

<150> VN1-2012-03153
<151> 2012-10-24

<150> DKPA 2013 70328
<151> 2013-06-18

<160> 4

<170> BiSSAP 1.2

<210> 1
<211> 495
<212> DNA
<213> Aeromonas Hydrophila

<220>
<221> source
<222> 1..495
<223> /mol_type="genomic DNA" /organism="Aeromonas Hydrophila"

<400> 1

```
ttcctgcaca tgatgtttgg cgtgccgacc gaagagtaca aggtcaaccc catcgtcgaa      60

cgcgccatgg accgcatctt caccctgcat gcggatcacg agcagaacgc ctccacctcc     120

accgtgcgtc tggccggctc gtccggtgcc aacccgttcg cctgtatcgc cgcggggatc     180

gcctccctgt ggggaccggc ccacggcggt gccaacgaag cctgcctcaa gatgctcgag     240

gagatcggct ccgtggatcg catccccgag tacatcgcca aggccaagga caagaacgat     300

ccgttccgtc tgatgggctt cggtcaccgg gtctacaaga accacgaccc ccgtgccacc     360

gtgatgcgcg aaacctgtca cgaggtactg accgagctgc agatcaagga tccgctgctg     420

gacgtggcca tggagctgga gcgcatcgcg ctgtccgacc cctacttcat cgagaagaag     480

ctctacccga acgtg                                                      495
```

<210> 2
<211> 504
<212> DNA
<213> Aeromonas Hydrophila

<220>
<221> source
<222> 1..504
<223> /mol_type="genomic DNA" /organism="Aeromonas Hydrophila"

<400> 2

```
gccgatatct gggttcgtta tcagcgaatg cgcggtcacc aggtgcactt tgtctgtgcc      60

gacgatgccc atggcacccc gatcatgctc aaggcccaac agctggggat caccccggaa     120

gagatgatcg cggcagtctc ccaggagcac caggccgact tcgccgggtt caacatcagt     180

tttgacaact atcactccac ccacagcgac gagaaccgtg agctggccgg gcttatctac     240

ggccgtctgc aggccggcgg caagatcaaa agccgcacca tctcccagct gttcgatccg     300

gaaaaatcca tgttcctgcc ggatcgtttc gtcaagggca cctgccccaa gtgcaagtcc     360

ccggagcagt atggcgacaa ctgcgacagc tgcggcgcta cctacagccc gaccgaactg     420

atcgatccga aatccgccgt ctccggcgct accccggtga tgaaggactc cgagcacttc     480

ttcttcgacc tgccgcagtt tgag                                           504
```

<210> 3
<211> 495
<212> DNA

<213> Aeromonas Hydrophila

<220>
<221> source
<222> 1..495
<223> /mol_type="genomic DNA" /note="sequences of the genes coding for gltA in biotype B (AL20215)" /organism="Aeromonas Hydrophila"

<400> 3

```
ttcctgcaca tgatgtttgg cgtgccgacc gaggagtaca aggtcaaccc catcgtcgaa      60

cgcgccatgg accgcatctt taccctgcat gcggaccacg agcagaacgc ctccacctcc     120

accgtgcgtc tggccggctc ctccggcgcc aacccgtttg cctgtatcgc cgcggggatc     180

gcctccctgt ggggaccggc ccacggtggt gccaacgaag cctgcctcaa gatgctcgag     240

gagatcggct ccgttgaccg cattcccgaa tacatcgcca aggccaagga caagaacgat     300

ccattccgtc tgatgggctt cggtcaccgg gtctacaaga accacgaccc ccgtgccacc     360

gtgatgcgcg aaacctgtca cgaggtactg accgagctgc agatcaagga tccgctgctg     420

gacgtggcca tggagctgga gcgcatcgcg ctctccgacc cctacttcat cgagaagaag     480

ctctacccga acgtg                                                     495
```

<210> 4
<211> 504
<212> DNA
<213> Aeromonas Hydrophila

<220>
<221> source
<222> 1..504
<223> /mol_type="genomic DNA" /note="DNA sequences of the genes coding for metG in biotype B (AL20215)" /organism="Aeromonas Hydrophila"

<400> 4

```
gccgatatct gggttcgtta tcagcgaatg cgcggtcacc aggtgcactt tgtctgtgcc      60

gacgatgccc acggcacccc gataatgctc aaggcccaac agctggggat caccccggaa     120

gagatgatcg cggccgtctc caaggaacac caggccgact tcgccgggtt caacatcagt     180

tttgacaact atcactccac ccacagcgac gagaaccgcg agctggcgga gctgatctac     240

ggccgtctgc aggccggcgg caagatcaag agccgcacca tctcccagct gttcgatccg     300

gaaaagtcca tgttcctgcc ggatcgcttc gtcaagggta cctgccccaa gtgcaagtcc     360

ccggagcagt atggcgacaa ctgtgacagc tgcggcgcca cctacagccc gaccgagctg     420

attgatccga aatccgccgt ctccggcgcc accccggtga tgaaggactc cgagcacttc     480

ttcttcgatc tgccgcagtt tgaa                                           504
```

**Description**

**Technical field of the invention**

It is an objective of the present invention to provide a composition, e.g. in form of an immunogenic composition or a vaccine, for the protection of fish, such as fish of the order Siluriformes, Perciformes and Cypriniformes against infection from *Aeromonas hydrophila.* More specifically, the inventors have surprisingly found that two different biotypes, biotype A and B, of *Aeromonas hydrophila* are capable of causing virulence in fish, such as fish of the order Siluriformes, Perciformes and Cypriniformes. Moreover, the inventors have surprisingly found that combining immunogenic amounts of two highly virulent *Aeromonas hydrophila* strains, one belonging to biotype A and one belonging to biotype B, e.g. in an immunogenic composition or in a vaccine, said combination surprisingly turned out to be capable of providing efficient prophylactic protection of fish, such as fish of the order Siluriformes, Perciformes and Cypriniformes against infection caused by *Aeromonas hydrophila.*

**Background of the invention**

In Santos Y. et al. 1987 the relationship between the phenotypic characteristics of certain *Aeromonas* species, including *Aeromonas hydrophilia,* and the virulence in rainbow trout (*Salmo gairdneri*) were studied by analyzing the biochemical, enzymatic and cell surface properties of several different *Aeromonas* strains isolated from fish culture systems. There is, however, no disclosure or suggestion of neither the specific pathogenic *Aeromonas hydrophila* biotypes A and B of the present invention nor of any fish vaccines based on said biotypes.

In Esteve C. et al. 1993 European eels were challenged both intraperitoneally and by waterborne exposure with certain strains of *Aeromonas hydrophilia.* On the basis of the challenge experiments the authors inter alia concluded that *Aeromonas hydrophila* acted as primary pathogens for European eels. There is, however, no disclosure or suggestion of neither the specific pathogenic *Aeromonas hydrophila* biotypes A and B of the present invention nor of any fish vaccines based on said biotypes.

In Chandran M. R. et al (2002), the Indian major carps *Catla catla, Labeo rohita* and *Cirrhinus mrigala* were immunised against *Aeromonas hydrophila* in field conditions.

In Phan et al. 2009, various aspects of the intensive aquaculture of striped catfish, *Pangasianodon hypophthalamus* in the Mekong delta in Vietnam were studied. It was inter alia reported that a consequence of intensified fish-farming was mortality rate of up to 30% during the early to mid-months of a production cycle. The authors also reported that the management of health on the catfish farms mainly involved chemical treatment, often with antibiotics and use feed additives (vitamin C). There is, however, no disclosure or suggestion of neither the specific pathogenic *Aeromonas hydrophila* biotypes A and B of the present invention nor of any fish vaccines based on said biotypes.

In M. E. Martino et al. 2011 the challenge within the art of establishing an unequivocal link between the *Aeromonas* genus and pathogenesis was disclosed as being extremely complicated, e.g due to the highly complex taxonomy of *Aeromonas.* The article furthermore discloses that only a small subset of strains containing genes for potential virulence factors seems to cause infection. Also disclosed were the challenges within the art of accurately discriminating between *Aeromonas* species. Envisaged was also that the microbial diversity (e.g. the phylogenic and taxonomic relationship) between the analyzed *Aeronomas* strains could be determined on the basis of multilocus sequence typing (MLST) and phenotypic characteristics. Amongst the *Aeromonas* strains tested were i.a. *Aeromonas hydrophila* collected from skin and kidney of e.g. Rainbow trout, Sea bream and Carp. On the basis of said metods, the authors were able to establish the taxonomic relationships among the analyzed *Aeromonas* strains and phenotypes and showed in general terms that *Aeromonas hydrophila* fit into the same phenogroup, described as the *Aeromonas hydrophila* complex. There is, however, no disclosure of neither the specific pathogenic *Aeromonas hydrophila* biotypes A and B of the present invention nor of any fish vaccines based on said biotypes.

In Pridgeon J. W. and Klesius P. H., 2011, attenuated vaccines based on the virulent and antibiotic-resistant "2009 West Alabama isolates" of *Aeromonas hydrophila* for use in channel catfish (*Ictalurus puncatus*) and Nile tilapia (*Orechromis niloticus*) were disclosed. The article also discloses that it is well known that *Aeromonas hydrophila* is very heterogeneous biochemically and serologically, which is considered as the biggest obstacle in developing effective commercial vaccine against *Aeromonas hydrophila.* Additionally, the article discloses that antibiotic-resistant *Aeromonas hydrophila* was virulence attenuated and, thus, considered to be suitable for use in fish vaccines. Both IP-injection and bath immersion were disclosed as possible routes of administration of the vaccine. However, no combination vaccine, involving two or more specific biotypes of *Aeromonas hydrophila,* was disclosed.

Studies of the exposure of fish to *Aeromonas hydrophila* are also described in WO 2010/099444, US 2012/258139, Crumlish M. et al. (2010), and Pridgeon J. W. et al (Vaccine 2011).

In Zheng W. et al., 2012, bacterial infection in grass carps (*Ctenopharyngodon idellus*) with four different virulent and antibiotic-resistant strains of *Aeromonas hydrophila* was studied. According to the article the four strains were identified as different *Aeromonas hydrophila* isolates using the ATB 32GN system, phylogenetic analysis, enterobacterial repetitive intergenic consensus-polymerase chain reaction (ERIC-PCR). The article further discloses that multi-infection of *Aeromonas hydrophila* strains may be an emerging threat in grass carp farming. However, even if infection with multiple strains of *Aeromonas hydrophila* in grass carps and in modern fish-farming in general was envisaged, the article does neither disclose nor suggest combined Aeromonas *hydrophila* based fish vaccines suitable for use in prophylactic treatment of said infections. In fact, the article envisages that treatment of the multi-infection of *Aeromonas hydrophila* could be conducted by use of common fishery drugs such as norfloxacin (norfloxacin is a broad-spectrum antibiotic that is active against both Gram-positive and Gram-negative bacteria).

Chinese patent application CN 101642567 discloses an inactivated fish vaccine based on a undefined pathogenic strains of *Aeromonas hydrophilia,* as well as the preparation of said vaccine, for the prevention and treatment of bacterial septicemia in carp. The vaccine is defined by the process in which the pathogenic - but undefined - *Aeromonas hydrophila* strains are extracted from infected carps and subsequently used for the preparation of a bacterial suspension for use in the vaccine. There is, however, no disclosure or suggestion of neither the specific pathogenic *Aeromonas hydrophila* biotypes A and B of the present invention nor of any fish vaccines based on the combination of said biotypes.

*State of the art - general considerations*

*Aeromonas hydrophila,* a gram negative motile bacillus widely distributed in the aquatic environment, is usually considered a secondary pathogen, but also can also be the causative agent of aeromonad septicaemia. Fish species affected are inter alia tilapia, catfish, goldfish, common carp and eel (Priodegon et al 2009). In recent years, some *Aeromonas hydrophila* strains have also been identified as highly virulent to carp (Zheng et al 2012) and catfish (Priodegon et al 2009).

In pangaisius farming in Vietnam, *Edwardsiella ictaluri* has been the major cause of mortality and according to Phan et al. (2009) 98% of the farms in the Mekong Delta in South Vietnam were affected. Pham et a.l also mention that 61% of the farms were affected by red spot. The mortality in the red spot disease group is most likely caused by *Aeromonas hydrophila.* Lately the disease identified in Vietnam as haemorrhage, causing red spots caused by *Aeromonas hydrophila* has increased causing heavy economic losses for the fish-farmers.

*Aeromonas hydrophila* is ubiquitous in nature and is even found in the intestinal tract of the fish. In natural situations in wild-life fish infections with *Aeromonas hydrophila* generally causes no major problems. However, with intensive fish-farming systems, whether these systems are outdoor ponds or indoor aquaria or tanks, other factors must be considered. The common occurrence of this disease relates to stress conditions or similar factors of the fish. It is commonly agreed within the field of aquaculture that fish are easily stressed when mishandled, overcrowded, transported under poor conditions, are on a poor level on nutrition, have poor water quality etc. This is all well-known within the art.

In all forms of intensive aquaculture, where single or multiple species are reared at high densities, infectious disease agents are easily transmitted between individuals. Due to the effectiveness of pathogen transportation in water and the high density of animals used in commercial large-scale fish-farming, pathogens quickly spread within a population of cultured fish.

It is well-known within the art that fish infected with *Aeromonas hydrophila* may have many different symptoms ranging from sudden death in otherwise healthy fish to lack of appetite, swimming abnormalities, pale gills, bloated appearance, skin ulcerations and exophthalmia. The skin ulcers may occur at any site on the fish and often are surrounded by a bright rim of red tissue. Other organs commonly affected with this disease include the gills, kidneys, liver, spleen, pancreas, and skeletal muscle. The symptoms vary since they are dependent upon a number of factors, particularly related to those of the diversity of the group of bacteria including the virulence of the organism, bur also factors related to the fish, as the resistance of the fish to infection, the presence or absence of bacteraemia or septicaemia as well as environmental factors. In effect, clinical outbreaks of *Aeromonas hydrophila* can potentially be economically disastrous for the fish farmer.

Therefore, traditionally, infectious diseases in fish-farmed fish have been prevented by the use of antibiotics. However, the use of antibiotics involves a multiplicity of potential problems.

After intensive, repeated medication using the same antibacterial agent, reduced efficacy is often seen after a period of time against the disease in question. As a consequence of the emergence of resistance, treatments must be repeated or new agents employed. However, antibiotic resistance is not only a problem of fish health. Resistance can develop in other bacteria in the sediments and in the water, thereby spreading to other organisms in the aquatic environment. The total burden of resistant bacteria people are subjected to is growing and may create new problems for human health.

More specifically, hitherto treatment of fish of the order Siluriformes, Perciformes and Cypriniformes against infection from *Aeromonas hydrophila* has been limited to two antibiotics, oxytetracycline, and potentiated sulfonamides. As mentioned, potential problems associated with any antibiotic therapy include inadequate dosage levels, overdosing, drug resistance by the bacteria and the chelating of calcium to hard water in the case of oxytetracycline used in a dip or bath.

It is therefore desirable that the use of antibiotics in the fish farming industry is reduced or preferably eliminated. In this regard it is particularly desirable to focus on this issue in geographical areas such as South East Asia, where the development of vaccines and general preventive health efforts have not come as far, and where considerable antibiotic use continues to be maintained.

*State of the art - general considerations - fish vaccines*

Already in the 1970s fish immersion vaccines based on formalin-inactivated broth cultures had proven to be effective against certain bacterial fish infections. The efficacy of these vaccines immediately resulted in a decline in the use of antibiotics. However, immersion vaccines turned out to have unsatisfactory effect against certain other fish pathogens. In effect, injectable vaccines containing adjuvants, typically oil-adjuvanted, were developed in the early 1990s.

Proper fish management with good hygiene and limited stress are key factors in the prophylaxis of infectious diseases and are also a necessity for the optimal effect of vaccines. Ideally, vaccines give long term protection against a multiplicity of infectious diseases, can be simply administered, are safe to use both for the fish and the administrator and are profitable. Moreover, compared with antibiotics, vaccines are environmentally friendly.

Several different types of fish vaccines are known within the art, including inactivated, live, recombinant and DNA vaccines. In general, there are three major modes for the application of vaccines: oral, immersion and injection. Whereas oral vaccines usually elicits short-term protection; immersion vaccines give better protection, whilst injectable vaccines give the best and longest lasting protection and are most cost-effective.

Nevertheless, today, vaccination is an integral part of most modern fish-farms and the use of antibiotics has been reduced considerably over the past two decades, in particular in Northern Europe and North America. In large parts of South America and South East Asia, however, antibiotics in commercial fish-farms is still intensely used which, in consequence, causes the above-outlined side-effects, e.g. in terms of resistance. Therefore, it is desirable to develop specific and effective vaccines against infectious diseases caused by e.g. antibiotic resistant bacteria in fish-farms in e.g. South East Asia.

**Summary of the invention**

The present invention relates to bacteria of *Aeromonas hydrophila* biotype A and B, of which representative isolates are deposited under the "Budapest Treaty on the international Recognition of the Deposit of Microorganisms for the Purposes of Patent Procedure of 28 April 1977".

Specifically, in a first aspect, the present invention relates to a composition comprising:

(i) an immunogenic amount of a live attenuated or inactivated Aeromonas hydrophila biotype A bacterium; and
(ii) an immunogenic amount of a live attenuated or inactivated Aeromonas hydrophila biotype B bacterium.

In a second aspect, the present invention relates to a composition comprising a bacterium of *Aeromonas hydrophila* biotype A, for use in preventing or reducing the incidence of septicemia in fish and/or for use in preventing or reducing the incidence of *Aeromonas hydrophila* infections in fish. The composition is co-administered with a composition comprising a bacterium of *Aeromonas hydrophila* biotype B.

In a third aspect, the present invention relates to a composition comprising a bacterium of *Aeromonas hydrophila* biotype B, for use in preventing or reducing the incidence of septicemia in fish and/or for use in preventing or reducing the incidence of *Aeromonas hydrophila* infections in fish, wherein said composition is co-administered with a composition comprising a bacterium of *Aeromonas hydrophila* biotype A.

In a fourth aspect, the present invention relates to a method of manufacturing a composition of the first aspect. The method comprises combining:

(i) an immunogenic amount of a live attenuated or inactivated *Aeromonas hydrophila* biotype A bacterium; and
(ii) an immunogenic amount of a live attenuated or inactivated *Aeromonas hydrophila* biotype B bacterium,

For the purposes of each of the first to fourth aspects of the present invention, an Aeromonas hydrophila biotype A bacterium is defined as having a Methionyl-tRNA synthetase (metG) gene comprising a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1.

In addition, for the purposes of the present invention, an Aeromonas hydrophila biotype B bacterium is defined as having a Methionyl-tRNA synthetase (metG) gene comprising a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

## Brief description of the figures

Figure 1 shows the maximum likelihood phylogenetic tree of 24 strains of *Aeromonas* using the genetic information employed in gltA and metG. Only bootstrap values above 50 are shown. The two conserved phylogenetic groups are labeled A and B. The sequence type (ST) is shown for all reference strains.

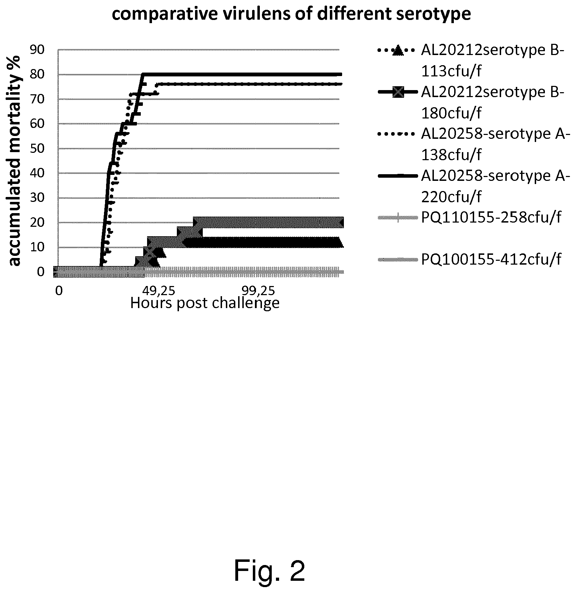

Figure 2 shows the %-accumulated mortality in pangasius (y-axis) and plotted against time (x-axis). Pangasius were injected intraperitoneally with biotype A, biotype B or another *Aeromonas hydrophila* strain (PQ100155). Mortality was monitored continuously.

The present invention will now be described in more detail in the following.

## Description of the invention

*Biotype A*

The inventors have found that immunogenic compositions based on *Aeromonas hydrophila* bacteria of biotype A protect fully against biotype A in challenge studies in fish of the order Siluriformes, Perciformes and Cypriniformes and provides satisfactory antibody response against biotype A.

In some embodiments, the *Aeromonas hydrophila* biotype A bacterium is represented by the bacterium deposited under the "Budapest Treaty on the international Recognition of the Deposit of Microorganisms for the Purposes of Patent Procedure of 28 April 1977" at the Health Protection Agency Culture Collections, Porton Down, Salisbury, Wiltshire, SP4 0JG, UK under accession number 12082901.

The inventors have also been able to establish both a genotypic and a phenotypic characterization of the above-defined strain of biotype A.

Further, it has been established that the above-defined bacterium of biotype A is capable of inducing specific clinical signs in infected fish. Thus, in some embodiments, the *Aeromonas hydrophila* biotype A bacterium has the ability to induce clinical signs of septicaemia, and optionally, mortality in pangasius (Pangasianodon hypophthalmus) when injected intraperitoneally in fish weighing 10-20 grams in an amount of 100-2000 cfu/fish and an injection volume of 0.1 ml.

Thus, in a further embodiment, the invention also relates to the above-defined bacterium of biotype A having the ability to induce clinical signs of septicaemia, and optionally, at least 50% mortality in pangasius (Pangasianodon hypophthalmus) within 2-5 days after intraperitoneal injection of said bacterium in fish weighing 10-20 grams in an amount of 100-2000 cfu/fish and an injection volume of 0.1 ml.

The above-defined ability of biotype A bacterium to induce mortality in pangasius, may be the ability of causing at least 50%, at least 60%, at least 70%, at least 80%, or such as at least 90% or 100% mortality. In particular embodiments, the ability to induce mortality is determined shortly after the bacteria have been isolated from a clinical outbreak, i.e. before extended storage.

In particular the fish according to the above embodiments may have a weight such as: 10-20 grams, 12-19 grams, 14-19 grams, 16-19 grams, 17-18 grams or 18 grams Additionally, the above embodiments may apply to fish starting at a weight of 5 grams and may further apply during the production cycle.

Moreover the amount of injected bacteria in the above embodiments may be: 100-2000 cfu/fish, 200-1500 cfu/fish, 300-1000 cfu/fish, 400-800 cfu/fish, 500-700 cfu/fish or less than 500 cfu/fish.

The inventors have also established that the above-defined bacterium of interest for the present invention may have a certain level of identity with two specific sequenced genes of the deposited strain of biotype A. Specifically, the above-defined bacterium of biotype A exhibited the following genotypic characteristics: it has a Methionyl-tRNA synthetase (metG) gene comprises a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1.

In particular, the above-defined bacterium of biotype A exhibits between 99.1% - 99.2%, 99.2% - 99.3%, 99.3% - 99.4%, 99.4% - 99.5%, 99.5% - 99.6%, 99.6% - 99.7%, 99.7% - 99.8%, 99.8% - 99.9%, 99.9%-100% identity to the sequence set forth in SEQ ID NO: 1.

Moreover, the above-defined bacterium of biotype A exhibits between 99.1% - 99.2%, 99.2% - 99.3%, 99.3% - 99.4%, 99.4% - 99.5%, 99.5% - 99.6%, 99.6% - 99.7%, 99.7% - 99.8%, 99.8% - 99.9%, 99.9%-100% identity to the sequence set forth in SEQ ID NO: 2.

As mentioned, the inventors have additionally established the serological characteristics of the above-defined bacterium of biotype A of interest for the present invention. The above-defined bacterium reacts with antiserum from fish immunized with the above-identified deposited bacteria of biotype A as determined in a direct agglutination test or slide agglutination test as described herein.

A direct agglutination test is used: serum from said fish may be added in serial dilutions in suitable receptacles such as wells in a microtiter plate, and an equal volume of bacteria is added to each receptacle or well. The bacteria may be prepared by transfering single colonies to TSB (Trypsic Soy Broth) followed by incubation at 28°C for 12 hours. The receptacle is incubated at suitable temperature; e.g. at 4°C and results are recorded, such as after 8 hours and 24 hours. A positive result is when the bacteria aggregate to form structures easily observed by the naked eye. A negative result is when the solution remains cloudy and does not form aggregates. The highest dilution of serum where agglutination is observed is recorded as the result.

Alternatively, the ability of a bacterium to react with antiserum from fish immunized with the deposited bacteria may be determined using a slide agglutination test as described herein. A slide agglutionation test may be performed by placing a drop of sterile saline on a clean slide. A single bacterial colony is then mixed with the water. One drop of fish serum is added, and the result recorded after 30 seconds. A positive result is when the bacteria aggregate to form structures easily observed by the naked eye. A negative result is when the solution remains cloudy and does not form aggregates.

In either test, serum is preferably used at a dilution of 1:2, such as of 1:4, 1:8, 1:16, 1:32, 1:64, 1:128, 1:256, 1:512, 1: 1024 or such as 1:2048.

*Biotype B*

The inventors have found that immunogenic compositions based on *Aeromonas hydrophila* bacteria of biotype B protect fully against biotype B in challenge studies in fish of the order Siluriformes, Perciformes and Cypriniformes and provide satisfactory antibody response against biotype B.

In some embodiments, the *Aeromonas hydrophila* biotype B bacterium is represented by the bacterium deposited under the "Budapest Treaty on the international Recognition of the Deposit of Microorganisms for the Purposes of Patent Procedure of 28 April 1977" at the Health Protection Agency Culture Collections, Porton Down, Salisbury, Wiltshire, SP4 0JG, UK under accession number 12082902.

The inventors have also been able to establish both a genotypic and a phenotypic characterization of the above-defined strain of biotype B.

Further, it has been established that the above-defined bacterium of biotype B is capable of inducing specific clinical signs in infected fish. Thus, in some embodiments, the *Aeromonas hydrophila* biotype B bacterium has the ability to induce clinical signs of septicaemia, and optionally, mortality in pangasius (*Pangasianodon hypophthalmus*) when injected intraperitoneally in fish weighing 10-20 grams in an amount of 100- 6000 cfu/fish and an injection volume of 0.1 ml.

Thus, in a further embodiment, the invention also relates to the above-defined bacterium of biotype B having the ability to induce clinical signs of septicaemia, and optionally, at least 50% mortality in pangasius (Pangasianodon hypophthalmus) within 2-5 days after intraperitoneal injection of said bacteria in fish weighing 10-20 grams in an amount of less 100-6000 cfu/fish and an injection volume of 0.1 ml.

The above-defined ability of biotype B bacterium to induce mortality in pangasius, may be the ability of causing at least 50%, at least 60%, at least 70%, at least 80%, or such as at least 90% or 100% mortality.

In particular the fish according to the above embodiments may have a weight such as: 10-20 grams, 12-19 grams, 14-19 grams, 16-19 grams, 17-18 grams or 18 grams. Additionally, the above embodiments may applyc to fish starting at a weight of 5 grams and may further apply during the production cycle.

Moreover the amount of injected bacteria in the above embodiments may be: 100-6000 cfu/fish, 200-5000 cfu/fish, 300-4000 cfu/fish, 400-3000 cfu/fish, 500-2000 cfu/fish, 600-1000cfu/fish or less than 500 cfu/fish.

The inventors have also established that the above-defined bacterium of interest for the present invention has a certain level of identity with two specific sequenced genes of the deposited strain of biotype B. Specifically, the above-defined bacterium of biotype B exhibited the following genotypic characteristics: it has a Methionyl-tRNA synthetase (metG) gene, that comprises a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

In particular, the above-defined bacterium of biotype B exhibited between 99.1% - 99.2%, 99.2% - 99.3%, 99.3% - 99.4%, 99.4% - 99.5%, 99.5% - 99.6%, 99.6% - 99.7%, 99.7% - 99.8%, 99.8% - 99.9%, 99.9%-100% identity to the sequence set forth in SEQ ID NO: 3.

Moreover, the above-defined bacterium of biotype B exhibited between 99.1% - 99.2%, 99.2% - 99.3%, 99.3% - 99.4%, 99.4% - 99.5%, 99.5% - 99.6%, 99.6% - 99.7%, 99.7% - 99.8%, 99.8% - 99.9%, 99.9%-100% identity to the sequence set forth in SEQ ID NO: 4.

As mentioned, the inventors have additionally established the serological characteristics of the above-defined bacterium of biotype B of interest for the present invention. The above-defined bacterium reacts with antiserum from fish immunized with the above-identified deposited bacteria of biotype B as determined in a direct agglutination test or slide agglutination test as described above.

*Composition*

The inventors have found that immunogenic compositions based on *Aeromonas hydrophila* bacteria of biotype A and immunogenic compositions based on *Aeromonas hydrophila* bacteria of biotype B and provides satisfactory antibody responses and protect fully against biotypes A and B, respectively, in challenge studies in fish of the order Siluriformes, Perciformes and Cypriniformes. The surprising finding in this regard resides in the realization by the inventors that by combining the two strains in one vaccine, said immune suppression seems to be neutralised when fish are immunized with two biotypes of *Aeromonas hydrophila* namely (i) an immunogenic amount of *Aeromonas hydrophila* biotype A bacteria represented by a strain deposited under the "Budapest Treaty on the international Recognition of the Deposit of Microorganisms for the Purposes of Patent Procedure of 28 April 1977" under accession number 12082901, and (ii) an immunogenic amount of *Aeromonas hydrophila* biotype B bacteria represented by a strain deposited under the "Budapest Treaty on the international Recognition of the Deposit of Microorganisms for the Purposes of Patent Procedure of 28 April 1977" under accession number 12082902. The *A hydrophila* strains are as described hereinbefore.

Thus, the invention relates to compositions comprising immunogenic amounts of the bacteria and/or antigenic and/or immunogenic material derived therefrom.

The invention provides a composition comprising:

(i) an immunogenic amount of a *Aeromonas hydrophila* biotype A bacterium;
and
(ii) an immunogenic amount of a *Aeromonas hydrophila* biotype B bacterium.

Having been able to establish the serological characteristics of the above-defined bacteria of biotype A and B, the invention, thus, relates to the above-defined composition characterised in that the immunogenic amount of said *Aeromonas hydrophila* bacterium is in a live attenuated, inactivated or killed form or any mixtures thereof.

Within the field of fish farming, administration of vaccines typically requires extensive handling of the fishes, e.g. in form of transferring the fishes to tanks with anaesthesia, injecting the fishes with vaccine and subsequently transferring of the fishes between tanks or ponds. These are only examples of activities relating to the vaccination program, which could potentially cause stress and increased mortality to farmed fish. Due to the large scale of modern aquaculture vaccination programs are costly, labor-intensive and capable of causing stress to the fish. For these reasons, it is desirable to provide fish vaccines against various infectious diseases preferably administered in form of e.g. combined, polyvalent vaccines containing several antigens capable of combating several infectious diseases.

In this context, the inventors have furthermore established that combining isolates of *Aeromonas hydrophila* belonging to biotype A and B with other antigen, such as, but not limited to, *Edwardsiella ictaluri, Edwardsiella tarda, Flavobacterium columnaris, Streptococcus iniae, Streptocossus agalactiae, Streptococcus dysgalactiae, Streptococcus parauberis,* in e.g. an immunogenic composition or a vaccine is capable of providing effective immunological responses in fish challenged with said antigens. It has *inter alia* been demonstrated that immunogenic compositions or vaccines in which antigens from (i) *Aeromonas hydrophila* biotype A and *Edwardsiella ictaluri* are combined and antigens from (ii) *Aeromonas* hydrophila biotype B and *Edwardsiella ictaluri* are combined protects against challenge with *Aeromonas hydrophila* belonging to biotype A and biotype B and *Edwardsialla ictaluri.*

Thus, in a still further aspect of the invention, the above-defined composition is characterized in that the immunogenic amount of said *Aeromonas hydrophila* bacterium is combined with one or more additional antigens, such as antigens derived from the bacteria *Edwardsiella ictaluri, Edwardsiella tarda, Flavobacterium columnaris, Streptococcus iniae, Streptocossus agalactiae, Streptococcus dysgalactiae, Streptococcus parauberis.*

It has furthermore been demonstrated that effective immunological effects in fish immunized, e.g. via an immunogenic composition or vaccine according to the present invention, requires a minimum amount of antigen.

Thus, in a still further embodiment of the invention, the above-defined composition is characterized in that (i) *Aeromonas hydrophila* bacteria are present in amounts $\geq 10^7$ bacteria/ml.

In a phylogenetic comparison study, the present inventors were able to demonstrate - on the basis of 24 strains (11 strains originating from PHARMAQ and 13 publicly available reference strains) - the phylogenetic relationship between biotype A and B of interest according to the present invention.

Thus, in some embodiments, the above-defined composition is characterized in that the DNA sequences for Methionyl-tRNA synthetase (metG) and Citrate synthase I (gltA) genes:

(i) in *Aeromonas hydrophila* biotype A bacteria are identical when compared internally, and
(ii) in *Aeromonas hydrophila* biotype B bacteria are identical when compared internally, and
(iii) when *Aeromonas hydrophila* biotype A and *Aeromonas hydrophila* biotype B are compared they display between 95-99.9% DNA sequence identity for gltA, and
(iv) when *Aeromonas hydrophila* biotype A and *Aeromonas hydrophila* biotype B are compared they display between 95-99.9% DNA sequence identity for metG, and
(v) when *Aeromonas hydrophila* biotype A and *Aeromonas hydrophila* biotype B are compared they display between 95-99.9% DNA sequence identity.

In this regard, under point (iii), 95-99.9% DNA sequence identity for gltA includes inter alia: preferably 96-99% DNA sequence identity for gltA, more preferably 97-98% DNA sequence identity for gltA and most preferably 97.4 % DNA sequence identity for gltA.

Under point (iv), 95-99.9% DNA sequence identity for metG includes inter alia: preferably 95.5-98% DNA sequence identity for metG, more preferably 96-97% DNA sequence identity for metG and most preferably 96.2 % DNA sequence identity for metG

Under point (v), 95-99.9% DNA sequence identity when gltA and metG are compared includes inter alia: preferably 95.5-98% DNA sequence identity when compared, more preferably 96-97% DNA sequence identity when compared and most preferably 96.8 % DNA sequence identity when compared.

As an additional embodiment the above-defined composition is in a dosage form suitable for intraperitoneal injection, e.g. in a volume of between 0,01 to 0,2 ml/dosage, more preferably between 0,03-0,15 ml/dosage, even more preferably between 0,05-0,1ml/dosage.

In a further additional embodiment of the invention the dosage of the above-defined composition is defined as comprising *Aeromonas hydrophila* bacteria in amounts $\geq 10^7$ bacteria/ml.

The inventors have shown that vaccines with antigen from biotype A protects against challenge with *Aeromonas hydrophila* belonging to serogroup A, but not against challenge with *Aeromonas hydrophila* isolates belonging to serogroup B. Vaccines with antigen from biotype B protects against challenge with *Aeromonas hydrophila* belonging to serogroup B, but not against challenge with *Aeromonas hydrophila* isolates belonging to serogroup A. If the vaccine contains antigen from both biotypes, i.e. biotype A and B of *Aeromonas hydrophila,* the vaccine protects against challenge with *Aeromonas hydrophila* isolates from both biotype A and B.

Furthermore, it has been shown that a combination vaccine comprising biotype A and biotype B is capable of effectively providing protection against strains tested from clinical outbreaks of e.g. *Aeromonas hydrophila* in e.g. South East Asia, such as clinical outbreaks in the Mekong delta in Vietnam.

Thus, in this context, as an additional aspect the above-defined composition is in form of an immunogenic composition such as a vaccine.

In a still further aspect, the invention relates to a composition as defined above for use in preventing or reducing the incidence of septicemia in fish/for use in preventing or reducing the incidence of *Aeromonas hydrophila* infections in fish, including inter alia fish of the order Siluriformes, Perciformes and Cypriniformes. If belonging to the order Siluriformes the fish is preferably pangasius (*Pangasianodon hypophthalmus*).

*Use*

The invention also relates to use of a composition as defined above for the manufacture of a medicament for preventing or reducing the incidence of septicemia in fish and/or for use in preventing or reducing the incidence of *Aeromonas hydrophila* infections in fish.

In specific embodiments the invention provide the use of a bacterium of *Aeromonas hydrophila* biotype A as described hereinabove in the manufacture of a composition for preventing or reducing the incidence of septicemia in fish/for use in preventing or reducing the incidence of *Aeromonas hydrophila* infections in fish, wherein said composition is co-administered with a composition comprising a bacterium of *Aeromonas hydrophila* biotype B.

In equally specific embodiments the invention provides the use of a bacterium of *Aeromonas hydrophila* biotype B as described hereinabove for the manufacture of a composition for preventing or reducing the incidence of septicemia in fish/for use in preventing or reducing the incidence of *Aeromonas hydrophila* infections in fish, wherein said composition is co-administered with a composition comprising a bacterium of *Aeromonas hydrophila* biotype A as described hereinabove.

Further embodiments provide a composition comprising a bacterium of *Aeromonas hydrophila* biotype A as described hereinbefore for use in preventing or reducing the incidence of septicemia in fish/for use in preventing or reducing the incidence of *Aeromonas hydrophila* infections in fish, wherein said composition is co-administered with a composition comprising a bacterium of *Aeromonas hydrophila* biotype B as described hereinbefore.

Still further embodiments provide a composition comprising a bacterium of *Aeromonas hydrophila* biotype B as described hereinbefore, for use in preventing or reducing the incidence of septicemia in fish/for use in preventing or reducing the incidence of *Aeromonas hydrophila* infections in fish, wherein said composition is co-administered with a composition comprising a bacterium of *Aeromonas hydrophila* biotype A as described hereinbefore.

In this context, the fish are preferably of the order Siluriformes, Perciformes and Cypriniformes. If belonging to the order Siluriformes the fish is preferably pangasius (*Pangasianodon hypophthalmus*).

*Method of manufacture*

The invention also relates to a method of manufacturing a composition as defined above, comprising combining

(ii) an immunogenic amount of a live attenuated or inactivated *Aeromonas hydrophila* biotype A bacterium; and
(iii) an antigenic and/or immunogenic material selected from an immunogenic amount of a live attenuated or inactivated *Aeromonas hydrophila* biotype B bacterium.

The example section provides the experimental background for the above-outlined aspects according to the present invention.

**Detailed description of the invention**

*Form of bacteria used in the immunogenic composition/vaccine*

The bacteria of the combination vaccine according to the invention may be present in a live attenuated form, in an inactivated form, e.g. as a bacterin or even in fragmented form. The important factor is this respect is that the immunogenic properties of the bacteria are still present.

The live attenuated bacteria have the advantage over bacterins that they can easily be given without an adjuvant. Moreover they self-replicate to a certain extent until they are stopped by the immune system, as a result of which a lower number of cells can be given. On the other hand, the immunogenic characteristics are also present on bacteria when these bacteria are in the form of a bacterin where bacterins inter alia have the advantage over live attenuated bacteria that they are very safe in use.

Therefore, in a preferred form of this embodiment, the invention relates to an immunogenic composition or vaccine wherein the *Aeromonas hydrophila* cells are inactivated.

Inactivation of the bacteria may be obtained by chemical or physical means. Chemical inactivation can be carried out by treatment of the bacteria by for example, but not limited to, treatment with enzymes, with formaldehyde (formalin), β-propiolactone or ethyleneimine or a derivative thereof, with organic solvent and/or detergent. Physiological inactivation can advantageously be carried out by subjecting the bacteria to energy-rich radiation, such as UV light, gamma irradiation or X-rays. These are inactivation means well-known in the art.

The present inventors have experienced that formaldehyde (formalin) inactivation is indeed a useful approach of inactivating biotype A and B of *Aeronomas hydrophila*. Therefore, in a preferred embodiment the bacteria are inactivated by addition of formaldehyde.

*Aeromonas hydrophila biotype A and B combined with other antigens*

The immunogenic composition or vaccine according to the present invention also encompasses combining *Aeromonas hydrophila* biotype A and B with other antigens such as, but not limited to, *Edwardsiella ictaluri, Edwardsiella trada, Flavobacterium columnaris, Strepotococcus iniae, Strepotocossus agalactiae, Streptococcus dysgalactiae, Strepotococcus parauberis.*

*Pharmaceutically acceptable carriers - adjuvants*

Vaccines are various preparations of antigens derived from specific pathogenic organisms that are rendered non-pathogenic. They stimulate the immune system and increase the resistance to disease from subsequent infection by the specific pathogen. A vaccine can be either water or oil based. Typically, injection vaccines are oil based as the oil provides adjuvant qualities. This means that the oil increases the effectiveness of the vaccine as well as the duration of the desired protection.

Oil adjuvants of the present invention suitable for use in water-in-oil emulsions are e.g. mineral oils or metabolisable oils. Metabolisable oils are e.g. vegetable oils, such as peanut oil and soybean oil, animal oils such as the fish oils squalane and squalene, and tocopherol and its derivatives.

Suitable adjuvants of the present invention are e.g. water-in-oil emulsions, oil-in-water emulsions and water-in-oil-in-water double emulsions. It is known in the art that especially inactivated vaccines, such as bacterins show improved immunogenicity when given as a water-in-oil emulsion.

Often, a vaccine or immunogenic composition is mixed with stabilizers, e.g. to protect degradation-prone proteins from being degraded, e.g. to enhance the shelf- life of the vaccine. Useful stabilizers of the present invention are i.a. carbohydrates e.g. sorbitol, mannitol, trehalose, starch, sucrose, dextran or glucose, proteins such as albumin or casein or degradation products thereof, and buffers, such as alkali metal phosphates, all known in the art.

In addition, the vaccine may be suspended in one or more pharmaceutically acceptable carrier(s).

Examples of pharmaceutically acceptable carriers that are suitable for use in a vaccine according to the invention are sterile water, saline, aqueous buffers such as PBS and the like.

Immunogenic compositions or vaccines according to the present invention, especially the immunogenic compositions vaccines comprising a bacterin, may in a preferred presentation also contain an immunostimulatory substance.

The immunogenic compositions or vaccine according to the invention may also comprise a so-called "vehicle". A vehicle is a compound to which the bacterium adheres, without being covalently bound to it. Such vehicles are i.a. bio-microcapsules, micro-alginates, liposomes and macrosols, all known in the art.

In addition, the immunogenic compositions or vaccine may comprise one or more suitable surface-active compounds or emulsifiers, all known in the art.

An extensive overview of adjuvants suitable for fish and shellfish vaccines is given in the review paper by Jan Raa (1996). Useful inorganic adjuvants will be known to the skilled artisan and are described JC Aguilar and EG Rodriguez, 2007, Vaccine 25, 3752 - 3762.

*Routes of administration (dosage forms)*

Many ways of administration, all known in the art, can be applied. The immunogenic compositions or vaccine according to the invention is preferably administered to the fish via injection, immersion, dipping or per oral. Especially immersion application and intraperitoneal injection are attractive ways of administration. As a preferred embodiment of the invention intraperitoneal injetion with a bacterin with an adjuvant is a very effective route of vaccination.

While other dosage forms may be contemplated, including solid dosage forms based on a fish feed or liquid forms for immersion, the dosage form according to the invention is preferably a liquid dosage form, more preferably a liquid dosage form for injection, such as intraperitoneal injection.

Below-outlined are several different routes and modes of vaccine administration according to the invention.

*Oral route*

Oral vaccination with antigen, wherein the vaccine is either mixed with the feed, coated on top of the feed (topdressed) or bio-encapsulated are known in the art. When antigens are to be incorporated in feed, the heat sensitivity of the antigen has to be considered. When vaccines are used as top dressing in feed, a coating agent is usually applied, either to prevent leaching of the antigen from the pellets or to prevent breakdown of the antigen in the acidic environment of the fish stomach.

Bio-encapsulation can be used where fish fry are to be vaccinated. For example, live feed, such as Artemia nauplii, copepods or rotifers, are incubated in a vaccine suspension after which they are fed to the fry. Since these live organisms are non-selective filter feeders, they will accumulate the antigen in their digestive tract and as such, transform themselves into living microcapsules.

*Immersion vaccine*

Skin epithelium and gills have mechanisms to protect fish in a broad as well as specific way. Immersion vaccination works on the ability of mucosal surfaces to recognize pathogens they had been in contact with. When fish are immersed in water containing the diluted vaccine, the suspended antigens from the vaccine may be adsorbed by the skin and gills. Then, specialized cells, such as antibody-secreting cells, present in the skin and gill epithelium will be activated and will protect the fish when fish are exposed to the live pathogen at a later stage. Other cells located in the epithelium of skin and gills, such as antigen presenting cells (macrophages/dendritic cells), also absorb vaccine antigens and transport them to specialized tissues where the systemic immune response builds up.

In immersion vaccination, there are two application methods: dip and bath. In dip vaccination, fish are immersed for a very short duration, usually 30 seconds, in a highly concentrated vaccine solution, usually 1 part vaccine product to 9 parts water. With bath vaccination, fish are exposed for a longer period, usually one to several hours, in a lower concentration of vaccine.

*Injection vaccination*

Light anaesthesia of fish is needed for injection vaccination. This decreases the stress due to vaccination, prevents mechanical injuries and helps the fish to recover faster from the handling.

Injection vaccines can be administered by intramuscular or intraperitoneal (in the abdominal cavity) injection, but the latter is by far the most common and preferable in according to the invention. As intraperitoneal injection vaccination involves depositing the vaccine in the abdominal cavity, it is important that the needle should penetrate the targeted abdominal wall of fish by 1 to 2 mm. Short needles might deposit the vaccine in the musculature and cause inflammation and a poor immune response.

It usually takes 2 or more weeks (usually around 400 degree days) before good immune protection is developed as a result of vaccination. Therefore, it is important not to stress the fish in the weeks following vaccination as stress is known to suppress the immune system.

Injection vaccination has a number of major advantages that makes it a preferred vaccination method. Injection vaccination provides for a long duration of protection, i.e., preferably for the whole production cycle and it allows for multiple antigens to be combined in a single vaccine given in a single administration. In addition, the fish farmer is assured that every fish in the population has received the vaccine and at the correct dose.

*Amounts/volume of bacteria used in the vaccine*

It will be understood that the vaccine according to the invention should contain antigens in amounts which are sufficient to elicit an immune response, preferably a protective response, after suitable administration (e.g. peritoneal injection) of the composition into a fish.

When intended for peritoneal injection the dosage form preferably has a volume of between 0.01 to 0.2 ml/fish, more preferably between 0.03-0.1ml/fish, even more preferably between 0.05-0.1ml/fish.

*Related genotypic and phenotypic characteristics*

In further embodiments, the bacteria used in the vaccine of the present invention are strains or isolates with genotypic characteristics which are related or similar to those of the deposited strains. By "related to or similar to" is meant that if a genetic typing according to example 2 is carried out, the strain would fall into the same category/group as the deposited strains. Falling into the same category/group as the deposited strain according to the present invention is defined as sharing more than 99% nucleotide sequence identity to the two genes according to example 2.

In still further embodiments the bacteria used in the vaccine of the present invention are strains or isolates with phenotypic characteristics which are related or similar to those of the deposited strains. By "related to or similar to" is meant that the phenotypically similar/related strain or isolate will react with antiserum prepared against the deposited strains.

The vaccine of the present invention is suitable for use in fish, e.g. in fish of the order Siluriformes, Perciformes and Cypriniformes, which includes but are not limited to the genus; Pangasius, Pangasionodon, Ictalurus, Oreochromis, Tilapia, and Cyprinus.

*Pangasius/Pangasionodon*

Pangasius is a type of catfish in the Order Siluriformes and in the genus Pangasiidae. Pangasiidae is native to the Mekong River and Chao Phraya basin in Thailand. The most important farmed fish for the international market is Pangasianodon hypophthalmus. They are often labeled in North America and Australia as "basa fish". In Europe, these fish are commonly marketed as "pangasius" or "panga". Pangasiidae is one of the fastest growing types of aquaculture in the world and the majority of farmed pangasius comes from just one species, *Pangasianodon hypohthalmus.* A small percentage is also Pangasius bocourti. Vietnam is the major producer of pangasius species.

The species in the genus Pangasianodon include but are not limited to *Pangasianodon hypophthalmus* and *Pangasianodon gigas.* The genus Pangasius include but are not limited to: *Pangasius bocourti, Pangasius conchophilus, Pangasius djambal, Pangasius elongatus, Pangasius humeralis, Pangasius kinabatanganensis, Pangasius krempfi, Pangasius kunyit, Pangasius larnaudii, Pangasius lithostoma, Pangasius macronema, Pangasius mahakamensis, Pangasius mekongensis, Pangasius myanmar, Pangasius nasutus, Pangasius nieuwenhuisii, Pangasius pangasius, Pangasius polyuranodon, Pangasius rheophilus, Pangasius sabahensis,* and *Pangasius sanitwongsei.*

*Channel catfish (Ictalurus punctatis)*

Ictalurus punctatus is also a species in the order Siluriformes. Also envisaged for the vaccine of the present invention it the channel catfish (*Ictalurus punctatus*) which is North America's most numerous catfish specie due to inter alia a rapid growth of aquaculture of this species in the United States over the last decade. Channel catfish is also produced in countries e.g. China.

*Tilapia group*

The tribe Tilapiini, is within the order Perciformes and the family Cichlidae (Cichlids). The tribe included the genera, Oreochromis, Sarotherodon, and Tilapia. The Tilapia group is one of the most commercially important fish in fish farming, because of their size, rapid growth, their herbivore nature, and palatability. Tilapias are the focus of major fish farming efforts, e.g. in China (being the largest tilapia producer in the world) followed by Egypt, Indonesia, Philippines, Thailand, Brazil, Vietnam and Taiwan.

The species of the tribe Tilapiini include but are not limited to *Oreochromis nicotilus, Oreomis aureus, Oreochromis hunter, Tilapia bakossiorum, Tilapia baloni, Tilapia bemini. Tilapia bilineata, Tilapia brevimanus,. Tilapia busumana, Tilapia buttikoferi, Tilapia bythobates, Tilapia cabrae, Tilapia cameronensis, Tilapia camerunensis, Tilapia cessiana, Tilapia coffea, Tilapia congica, Tilapia dageti. Tilapia deckerti, Tilapia discolour, Tilapia flava, Tilapia guinasana* (Otjikoto Tilapia). *Tilapia guineensis* (Guinean tilapia), *Tilapia gutturosa, Tilapia imbriferna, Tilapia ismailiaensis, Tilapia jallae, Tilapia joka. Tilapia kottae, Tilapia louka, Tilapia margaritacea, Tilapia mariae* (Black mangrove cichlid), *Tilapia nyongana. Tilapia rendalli* (Blue tilapia), *Tilapia rheophila, Tilapia ruweti* (Okavango tilapia), *Tilapia snyderae, Tilapia sparrmanii* (Banded bream), *Tilapia spongotroktis, Tilapia tholloni. Tilapia thysi, Tilapia walteri, Tilapia zillii* (Cichlid).

*Carp*

Carp are various species of freshwater fish of the family Cyprinidae and the order Cypriniformes, a very large group of fish native to Europe and Asia. The carp species include inter alia but are not limited to: *Hypophthalmichthys molitrix* (silver carp), *Cyprinus carpio* (common carp), *Ctenopharyngodon idella* (grass carp), *Hypophthalmichthys nobilis* (bighead carp), *Carassius carassius* (Cruian carp), *Cyprinus catla* (Catla carp), *Cirrhinus cirrhosus* (Mrigal carp), *Mylopharyngodon piceus* (Black carp), *Cirrhinus molitorella* (Mud carp) and *Labeo rohita* (Roho).

**Definitions**

*Aeromonas hydrophila*

*Aeromonas hydrophila* is a gram negative rod shaped bacterium of size 0.3 to 1 micrometer in width, and 1 to 3 micrometers in length. *Aeromonas hydrophila* is generally known to be avirulent or secondary pathogen in fish and is mainly found in areas with a warm climate. This bacterium can be found in fresh or brackish water and is often resistant to most common antibiotics. *Aeromonas hydrophila* is known to cause disease in many species of fish such as tilapia (*Oreochromis niloticus*), catfish (*Clariasbatrachus, Ichtalurus punctatus*), goldfish (*Crassius auratus*), common carp (*Cyprinus carpio*), eel (*Anguilla anguilla*) and rainbow trout (*Oncorhynchus mykiss*) (Santos et al 1988, Pridgeon and Klesius 2011, Esteve et al 1994).

*Isolated*

When used in relation to the bacteria of the present invention, the term "isolated" refers to the bacteria when separated from their natural environment, such as the cells and tissues from their piscine host. In particular, the term "isolated" refers to a culture, such as a pure culture of the bacteria derived from a tissue sample from an infected host.

*Combination vaccines/polyvalent vaccines*

Combination vaccine/polyvalent vaccine is defined as a vaccine that consists of two or more separate immunogens combined into a single product.

Whereas monovalent vaccines are designed to immunize against a single antigen or single microorganism, polyvalent combination vaccines are capable of providing immunity against a plurality of different diseases. In general, monovalent vaccines provides increased immunity against the relevant antigen compared to a polyvalent vaccine. This can *inter alia* be explained by the fact that in polyvalent vaccines interference can occur between the different biotypes when they are generating an immune response in the vaccinated fish whereas in the monovalent vaccine this does not occur.

*Clinical outbreaks*

Clinical outbreak refers to epidemiological study term used in epidemiology to describe an occurrence of disease greater than would otherwise be expected at a particular time and place.

*Motile aeromonad septicaemia (septicaemia disease)*

Septicemia (also septicaemia or septicaemia) refers to the presence of pathogenic organisms in the bloodstream, leading to sepsis.

Septicaemia disease caused by *Aeromonas hydrophila* or motile aeromonad septicaemia is one of the most common diseases in Vietnam pangasius culture. At Mekong delta, motile aeromonad septicaemia can occur throughout the year, especially causes significant loss by season changing from wet to dry or by stressor such as handling, transportation, high level of nitrite, ammonia etc. Clinical signs of motile aeromonad septicaemia are the haemorrhages caused by haemolysin from the bacteria.

External signs observed from most of affected fish were swollen head, exophthalmia, reddening of fish's surface, haemorrhage of anus and fins. Ulcerative skin lesions can also be often observed from infected fish with necrosis of fins or tail (fin rot).

Internally there may be an excess of clear or red-tinged fluid in clinical dropsy, but in most case the principal feature is hyperaemia of viscera with bright red colour. The spleen is visibly enlarged, rounded, and cherry red. The enlarged kidney has undergone liquefactive necrosis and when it is incised necrotic fluid oozes out.

The inventors have shown that *Aeromonas hydrophila* is a very high virulent pathogen to pangasius fish. In an experiment, challenge doses in a range of 100 to 2000 cfu biotype A/fish and challenge doses in a range of 100 to 6000 cfu biotype B/fish caused 60 to 100% mortality of fish within 2 days. The clinical disease signs of Motile aeromonad septicaemia were observed and *Aeromonas hydrophila* were re-isolated from dead fish.

In the field, sudden changes of temperatures, or handling or transfer of fish, can lead to losses with involvement of *Aeromonas hydrophila.* Motile aeromonad septicaemia could cause losses throughout the culture period, outbreaks might occur again and again in a particular pond. During the outbreak period, the cultured fish might be infected by other pathogens resulting in heavy loss to the farmer.

*Biotype*

The bacteria used in e.g. the composition or the vaccine of the present invention are strains or isolates with genotypic characteristics which are related or similar to those of the deposited strains. By "related to or similar to" is meant that if a genetic typing according to example 2 is carried out on the genotypically similar/related strains or isolates they would fall into the same group as the deposited strains, i.e. having at least 99% identity with the SEQ ID No. 1 and 2 (biotype A) or with SEQ ID No. 3 and 4 (biotype B).

Moreover, the bacteria used in e.g. the composition or vaccine of the present invention are strains or isolates with phenotypic characteristics which are related or similar to those of the deposited strains. By "related to or similar to" is meant e.g. that the phenotypically similar/related strain or isolate will react with antiserum prepared against the deposited strains.

*An immunogenic amount*

An immunogenic amount of *Aeromonas hydrophila* cells is the amount of antigen necessary to induce an immune response that is at least capable of reducing the severity of the disease and/or the incidence of infection, compared to non-vaccinated fish.

*Sequence identity*

In the context of the present invention, the term "sequence identity" indicates a quantitative measure of the degree of homology between two nucleotide sequences. If the two sequences to be compared are not of equal length, they must be aligned to give the best possible fit, allowing the insertion of gaps or, alternatively, truncation at the ends of the nucleotide sequences. The sequence identity can be calculated as $\frac{(N_{ref} - N_{dif})100}{N_{ref}}$, wherein Ndif is the total number of non-identical residues in the two sequences when aligned and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

With respect all embodiments of the invention relating to nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). For amino acid sequence alignments the settings are as follows: Alignment=3Dfull, Gap Open 10.00, Gap Ext. 0.20, Gap separation Dist. 4, Protein weight matrix: Gonnet.

Alternatively, nucleotide sequences may be analysed using programme DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well established method that finds the optimal local alignment of two sequences. The other algorithm, ParAlign, is a heuristic method for sequence alignment; details on the method are published in Rognes (2001). Default settings for score matrix and Gap penalties as well as E-values may be used.

*Genotypic characteristics*

The term "genotypic characteristics" refers broadly to the composition of one or more parts of an individual's genes or genome, and is always reflected in the individuals DNA sequence.

*Phenotypic characteristics*

The term "phenotypic characteristics" refers in the present application to one or more observable properties of an organism that are produced by interaction of the inherited genotype of the individual, and/or non-hereditary environmental variation (e.g. variation in transcription, mobile DNA and plasmids, genomic rearrangement etc.). In the context of the present invention, the term "related phenotypic characteristics" include any of the following characteristics: size, shape. "Phenotypic characteristics" further include the ability to induce any of the clinical signs of *Aeromonas hydrophila* in fish, including gross pathological signs as described in the present application. "Phenotypic characteristics" also include the ability of the bacteria to display the same antigens as the deposited strains, i.e. reacting to antisera raised against the deposited strains.

*A pharmaceutically acceptable carrier*

A pharmaceutically acceptable carrier can be water or a buffer, or an emulsion such as e.g. an oil-in-water or water-in-oil emulsion.

*Water-in-oil em ulsions*

Emulsions comprising water and oil are generally referred to as either water-in-oil emulsions, oil-in-water or water-in-oil-in-water emulsions. Whether an emulsion turns into a water-in-oil emulsion or an oil-in-water emulsion depends on the volume fraction of both phases and on the type of emulsifier. Generally, emulsifiers stabilize an emulsion.

*Pathology*

*Aeromonas hydrophila* it is virulent to many organisms and, after having entered the body the host, it travels through the bloodstream to the first available organ. *Aeromonas hydrophila* is considered to be an opportunistic pathogen, meaning it rarely infects healthy individuals. *Aeromonas hydrophila* is widely considered a fish pathogen. When infected with *Aeromonas hydrophila,* fish can develop ulcers, tail rot, fin rot, and hemorrhagic septicemia. Hemorrhagic septicaemia causes lesions that lead to scale shedding, hemorrhages in the gills and anal area, ulcers, exophthalmia, and abdominal swelling.

*Phylogeny*

Phylogeny deals with the evolutionary relationship between organisms. This is usually done by analyzing the DNA (or translated protein) sequence of one or several conserved genes (usually a housekeeping gene that evolves slowly) in the organisms of interest. The gene has to be present in all the organisms that are being analyzed. The most "famous" gene applied for this purpose is the 16S rRNA gene, which is highly conserved between all bacteria. However, due to its very high level of conservation it is usually not suitable for comparing different strains within the same species. Consequently other less conserved housekeeping genes are used to differentiate between similar bacterial strains. Also, by combining the evolution of several housekeeping genes at the same time, the phylogenetic prediction becomes more robust. Multilocus sequence typing (MLST), also known as Multilocus sequence analysis (MLSA), does this by combining the sequence information from e.g. 5 to 9 genes. Phylogenetic relationships are usually represented as phylogenetic trees generated by complex algorithms using the organisms DNA sequence as input.

*Multilocus sequence typing (MLST)*

MLST is a technique in molecular biology for the typing of multiple loci. The procedure is capable of characterizing isolates of microbial species using the DNA sequences of internal fragments of multiple housekeeping genes, i.e. constitutive genes that are transcribed at a relatively constant level. The characterization is based on differences in the sequences of these housekeeping genes.

*Slide agglutination tests*

Agglutination is the clumping together in suspension of e.g. antigen-bearing microorganisms in the presence of specific antibodies.
A slide agglutination test is a test in which a certain amount of concentrated antigen and e.g. fish serum are mixed on a slide and allowed to react for a specified period, after which the presence of agglutination is determined.

*Relative Percent Survival (RPS)*

RPS expresses the percentage of fish, which would have died from a given disease if not protected against it, i.e. the proportion of fish saved due to vaccination. An economically acceptable, successful vaccination should exceed a RPS value of 60%, meaning that in case of a disease outbreak the unvaccinated fish against this particular disease would suffer at least a three-fold mortality loss than the vaccinates.

$$RPS = [1-(\% \text{ of dead fish in vaccinated group}/\% \text{ of dead fish in control group})]*100$$

It should be noted that embodiments and features described in the context of one of the aspects of the present invention also apply to the other aspects of the invention.
The invention will now be described in further details in the following non-limiting examples.

**Example 1 :** Isolation of *Aeromonas hydrophila* from pangasius.

*Aeromonas hydrophila* causes a disease in pangasius called septicaemia disease or motile aeromonad septicaemia. This is one of the most common diseases in Vietnamese pangasius culture. In the Mekong River, motile aeromonad septicaemia outbreaks are more common when the seasons change from wet to dry, and/or when the fish are stressed by e.g. handling, transportation, high level of nitrite or ammonia or other types of environmental stress.
Clinical signs of motile aeromonad septicaemia are haemorrhages caused by haemolysin. External signs usually observed in affected fish are swollen head, exophthalmia, reddening of the surface of the fish and haemorrhages of anus and fins. Ulcerative skin lesions can also be often observed from infected fish with necrosis of fins or tail (fin rot).
Internally there may be an excess of clear or red-tinged fluid in clinical dropsy, but in most case the principal feature is hyperaemia of viscera resulting in a bright red colour. The spleen is visibly enlarged, rounded, and cherry red. The enlarged kidney has undergone liquefactive necrosis and when it is incised necrotic fluid oozes out.
To isolate *Aeromonas hydrophila,* a sterile bacteriology loop was inserted into the kidney or spleen of the fish and streaked directly onto for example tryptic soy agar plates. The *Aeromonas hydrophila* appeared as round colonies of creamy white colour. *Aeromonas hydrophila* is a gram negative rod shaped bacterium of size 0.3 to 1 micrometre in width, and 1 to 3 micrometres in length.

**Example 2:** Phylogeny of *Aeromonas hydrophila*

To classify the *Aeromonas hydrophila* isolates obtained by the method described in example 1, a simplified MLST-scheme was used which was based on Martino et al., 2011, employing the genetic information within the Methionyl-tRNA synthetase (metG) and Citrate synthase I (gltA) genes for phylogenetic predictions.
PCR was performed using primers described in Martino *et al.,* 2011. DNA sequences were assembled; quality checked and trimmed using Vector NTI® software. External reference sequences were retrieved from http://pubmlst.org/aeromonas, including all *Aeromonas hydrophila* strains from Martino *et al.,* 2011, and some additional *Aeromonas* species for reference purposes (see table below for complete strain information). Sequence alignments and phylogenetic predictions were performed in MEGA5, as described in Tamura *et al.,* 2011, with the following parameters:

Statistical Method: Maximum Likelihood (100 Bootstrap Replications)
Substitution Model: Tamura-Nei model
Rates: Gamma distributed with Invariant sites (16 Discrete Gamma Categories)

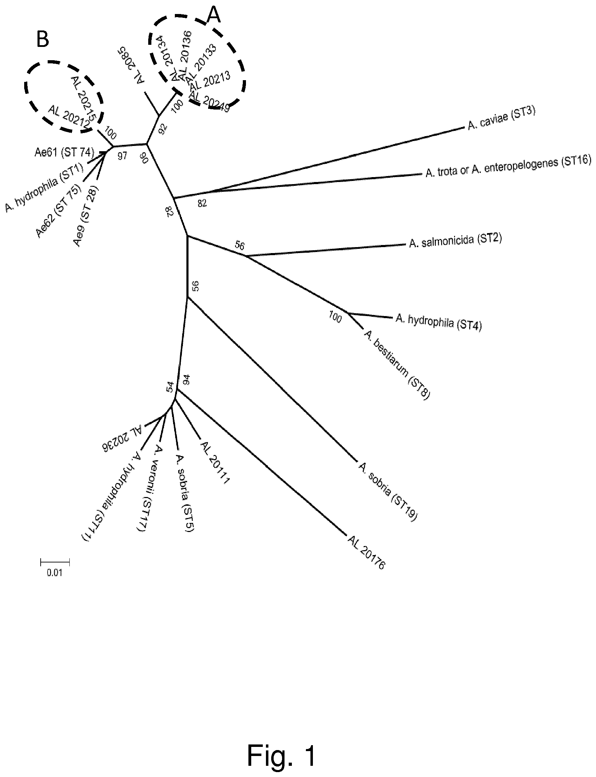

Upon analysis of the DNA sequences for metG and gltA the inventors have defined two distinct and separate phylogenetic groups for the strains analysed. For simplicity the two groups have been labelled A and B in figure 1. Strains within these two groups are identical when compared internally, however when compared to strains in the other group they display 97,4 % DNA sequence identity for gltA, 96,2 % DNA sequence identity for metG and 96,8 % DNA sequence identity when combined (gltA + metG).

| | Strain | Sequence type* | Source |
|---|---|---|---|
| **PHARMAQ strains** | AL 2 085 | - | Pangasius, Vietnam |
| | AL 20 111 | - | Fish, Costa Rica |
| | AL 20 133 | - | Pangasius, Vietnam |
| | AL 20 134 | - | Pangasius, Vietnam |
| | AL 20 136 | - | Pangasius, Vietnam |
| | AL 20 176 | - | Tilapia , Malaysia |
| | AL 20 212 | - | Pangasius, Vietnam |
| | AL 20 213 | - | Pangasius, Vietnam |
| | AL 20 215 | - | Pangasius, Vietnam |
| | AL 20 236 | - | Fish, Brazil |
| | AL 20 249 | - | Pangasius, Vietnam |
| **Reference / type strain**\*\* | *Aeromonas hydrophila*, ATCC 7966 | ST1 | Milk, UK |
| | *Aeromonas hydrophila*, CECT 398 | ST11 | Human feces of a child with diarrhea, USA |
| | *A. trota* or *A. enteropelogenes*, CECT 4255 | ST16 | Human feces, India |
| | *A. veronii bv. Veronii*, CECT 4257 | ST17 | Sputum of drowning victim, USA |
| | *A. sobria*, CECT 4245 | ST19 | Fish, France |
| | *A. salmonicida subsp. salmonicida*, NCIMB 1102 | ST2 | Atlantic salmon, UK |
| | *Aeromonas hydrophila*, Ae 9 | ST28 | Crayfish / hemolymph, Italy |
| | *A. caviae*, NCIMB 882 | ST3 | Goldfish, Argentina |
| | *A. bestiarum / Aeromonas hydrophila*, NCIMB 1134 | ST4 | Rainbow trout , UK |
| | *A. sobria*, NCIMB 75 | ST5 | Diseased freshwater fish, Germany |
| | *Aeromonas hydrophila*, Ae 61 | ST74 | Flathead gray mullet, Italy |
| | *Aeromonas hydrophila*, Ae 62 | ST75 | Sea bream, Italy |
| | *A. bestiarum*, DSM 13956 | ST8 | Infected fish, France |

**Table 1** : Information on all 24 strains included in the phylogenetic comparison is shown above, consisting of 11 strains originating from PHARMAQ and 13 reference strains. *as defined at the Web-based MLST sequence database (http://pubmlst.org/aeromonas). **table modified from Martino *et al.,* 2011.

**Example 3a** Virulence of different *Aeromonas hydrophila* strains isolated from fish

*Aeromonas hydrophila* biotype A (AL20258) biotype B (AL20212) and PQ110155 (neither biotype A nor B) was isolated as described in example 1, and propagated in TSB for 24 hours. Fish (*Pangasianodon hypophthalmus*) of 18g in weight, kept in fresh water at 28°C was injected 0.1 ml of two different concentrations of the bacteria suspension (less than 500cfu/fish). Mortality was observed continuously after the challenge. *Aeromonas hydrophila* biotype A and B induced rapid mortality, while PQ110155 was avirulent. With the challenge doses used, the fish injected with biotype A reached higher mortality than compared with biotype B. The dead fish all showed symptoms of Aeromonas septicemia.
This experiment shows that *Aeromonas hydrophila* of biotype A and B are virulent for pangasius and can be a primary pathogen for pangasius.

**Example 3b** Virulence of different *Aeromonas hydrophila* strains isolated from fish

Pangasius of approx. 12,5g were kept in fresh water at 27°C. Different isolates of *Aeromonas hydrophila* biotype A or B were used to challenge parallel groups of 20 fish in separate tanks by intraperitoneal injection. Mortalities were counted daily and accumulated mortality calculated. Fish in all tanks showed clinical signs of motile aeromonad septicaemia. Using doses ≤ 6000 bacteria/fish, all strains induced rapid, high mortality in the fish.
This experiment shows that *Aeromonas hydrophila* strains of both biotype A and B are virulent and causes mortality in pangasius. Some strains give mortality with challenge doses ≤1000 bacteria/fish, while others give high mortality with slightly higher challenge doses. *Aeromonas hydrophila* can be a primary pathogen for pangasius.

**Table 3:** Different strains of *Aeromonas hydrophila* from either biotype A or B were used to challenge pangasius of approx. 12.5 grams. % accumulated mortality in each tank was calculated.

| Challenge strain | Biotype | Challenge dose (cfu) | % mortality tank 1 | % mortality tank 2 |
|---|---|---|---|---|
| VH18 | A | 733 | 95 | 95 |
| AL 20258 | A | 489 | 90 | 95 |
| AL 20257 | A | 500 | 100 | 100 |
| AL 20212 | B | 5972 | 100 | 90 |
| AL 20215 | B | 1220 | 75 | 80 |
| AL 20 213 | A | 600 | 90 | 100 |

**Example 4:** Serology of *Aeromonas hydrophila* isolates from pangasius.

200 Pangasius fish (*Pangasionodon hypophthalmus*), 30 gram in average weight, were divided into 2 groups and vaccinated with a monovalent vaccine of *Aeromonas hydrophila,* based on the strain AL20136 = biotype A or based on the strain AL 20212 = biotype B.
The vaccines were formulated as water in oil emulsion vaccines, and each fish were injected 0.1 ml vaccine intraperitoneally. Blood samples from fish vaccinated with the respective vaccines were collected 4 weeks post vaccination. The blood was kept cool (18-20°C) and allowed to clot, then centrifuged at 6000 rpm for 5 minutes where after serum was collected. The sera were used in slide agglutination tests and direct agglutination tests in 96 well plates.
Preparation of the bacteria used in the tests:

The bacteria had been isolated from pangasius as described in example 1. They were recoverd on TSA (Tryptine Soya Agar) from frozen stocks and incubated at 28°C for 12 hours. After incubation on TSA, single colonies were transfered to TSB (Trypsic Soy Broth) then incubated at 28°C for 12 hours.

The bacterial cultures were centrifuged at 3500 rpm for 15 minutes, the medium was removed and the bacteria washed once and resuspended in sterile saline. The bacterial suspension was inactivated with 1% formalin and stored at 4°C until use.

Agglutination tests - 96 well plate test:

A direct agglutination test was used to examine the cross reaction between sera collected from the two different vaccinated fish groups and 24 different strains of *Aeromonas hydrophila* collected from pangasius.

The serum was diluted twofold in the plate, and an equal volume of bacteria prepared as described was added to each well. Positive and negative controls were included. Plates were left at 4°C and results recorded after 8 hours and 24 hours. A positive result is when the bacteria aggregate to form structures easily observed by the naked eye. A negative result is when the solution remains cloudy and does not form aggregates. The number of the well with the highest dilution of serum where agglutination is observed is recorded as the result. The corresponding dilution of the antiserum is as follows:

**Table 4:** dilution of the antiserum in the respective wells

| Well | Dilution of antiserum |
|------|----------------------|
| 1 | Undiluted |
| 2 | 1:2 |
| 3 | 1:4 |
| 4 | 1:8 |
| 5 | 1:16 |
| 6 | 1:32 |
| 7 | 1:64 |
| 8 | 1:128 |
| 9 | 1:256 |
| 10 | 1:512 |
| 11 | 1:1024 |
| 12 | 1:2048 |

Slide agglutination test:

The following procedure was used. One drop of sterile saline was put on a clean slide. A single colony from a TSA plate was picked and mixed with the water. One drop of fish serum was added, and the result recorded after 30 seconds. A positive result is when the bacteria aggregate to form structures easily observed by the naked eye. A negative result is when the solution remains cloudy and does not form aggregates.

Pangasius were vaccinated with water-in-oil vaccines containing antigen from *Aeromonas hydrophila* biotype A or B. Blood samples were taken from vaccinated fish four weeks post vaccination. When the fish are vaccinated, they will respond to the vaccine and produce specific antibodies to the antigen in the vaccine. The ability of the fish serum to agglutinate *Aeromonas hydrophila* biotype A or biotype B bacteria elucidates how similar the biotypes are. If the biotypes are similar, the serum will agglutinate them at the same level.

The results show that serum from fish vaccinated with biotype A agglutinate biotype A strains much better than biotype B strains and vice versa. This confirms that biotype A and biotype B represents two different biotypes of *Aeromonas hydrophila.* When antiserum is generated against one biotype, the antiserum does not react equally well with the other biotype and vice versa.

### 4 weeks post vaccination

| | Vaccine | AL 20136/ biotype A | |
|---|---|---|---|
| | *Aeromonas hydrophila* strain tested | AL 20136 (biotype A) | AL 20212 (biotype B) |
| Individual fish | 1 | 0 | 0 |
| | 2 | 5 | 0 |
| | 3 | 8 | 2 |
| | 4 | 6 | 4 |
| | 5 | 0 | 0 |
| | 6 | 9 | 0 |
| | 7 | 6 | 0 |
| | 8 | 8 | 5 |
| | 9 | 5 | 0 |
| | 10 | 7 | 0 |
| | **Average** | 5.4 | 1.1 |

**Table 5a**: Results from agglutination in a 96 well plate test. Serum from fish vaccinated with biotype A. Serum from 10 fish was tested. The well with the highest dilution of serum displaying agglutination is recorded as the result for the test.

| | | 4 weeks post vaccination | |
|---|---|---|---|
| | Vaccine | AL 20212/ biotype B | |
| | *Aeromonas hydrophila* strain tested | AL 20136 (biotype A) | AL 20212 (biotype B) |
| Individual fish | 1 | 3 | 6 |
| | 2 | 0 | 8 |
| | 3 | 0 | 6 |
| | 4 | 0 | 0 |
| | 5 | 0 | 5 |
| | 6 | 2 | 0 |
| | 7 | 5 | 8 |
| | 8 | 0 | 8 |
| | 9 | 0 | 2 |
| | 10 | 3 | 5 |
| | **Average** | 1.3 | 4.8 |

**Table 5b**: Results from agglutination in a 96 well plate test. Serum from fish vaccinated with biotype B. Serum from 10 fish was tested. The well with the highest dilution of serum displaying agglutination is recorded as the result for the test.

*Aeromonas hydrophila*

Several other strains isolated from pangasius were then tested for their ability to agglutinate with serum from fish vaccinated with either biotype A or biotype B vaccines. This was done with a slide agglutination test.
Results from the slide agglutination test:

Table 6: Different *Aeromonas* strains isolated from pangasius were tested by direct agglutination test as described. += agglutination observed. -= agglutination not observed.

| No | ID | Anti biotype A | Anti biotype B | Serogroup |
|---|---|---|---|---|
| 1 | PQ110255 | + | - | A |
| 2 | AL 20136 | + | - | A |
| 3 | AL 20215 | - | + | B |
| 4 | AL 20262 | + | - | A |
| 5 | AL 20255 | + | - | A |
| 6 | PQ110466 | + | - | A |
| 7 | AL 20257 | + | - | A |

(continued)

| No | ID | Anti biotype A | Anti biotype B | Serogroup |
|---|---|---|---|---|
| 8 | PQ110098 | + | - | A |
| 9 | PQ110458 | + | - | A |
| 10 | PQ110445 | + | - | A |
| 11 | AL 20258 | + | - | A |
| 12 | PQ120160 | + | - | A |
| 13 | AL 20212 | - | + | B |
| 14 | PQ110367 | + | - | A |
| 15 | PQ110488 | + | - | A |
| 16 | PQ110440 | + | - | A |
| 17 | AVH74 | + | - | A |
| 18 | PQ110501 | - | - | NONE |
| 19 | PQ110503 | + | - | A |
| 20 | PQ110345 | + | - | A |
| 21 | AL 20263 | + | - | A |
| 22 | PQ120604 | + | - | A |
| 23 | PQ110490 | + | - | A |
| 24 | PQ110228 | + | - | A |

The results show that most strains isolated from clinical outbreaks in Vietnam of *Aeromonas* in pangasius belongs to biotype A or B.

**Example 5:** Cross protection between biotype A and biotype B of *Aeromonas hydrophila*

Vaccines (inactivated bacteria in aqueous solution or water in oil emulsions) with different isolates of *Aeromonas hydrophila* belonging to biotype A or B were made and pangasius immunized by intraperitoneal injection of 0,05ml of said vaccines. The concentration of antigen was $\geq 10^7$ bacteria/ml. The pangasius were held under standard conditions in fresh water at $28 \pm 2°C$. 2 to 20 weeks after vaccination the fish were challenged by injecting 1000-10000 cfu of *Aeromonas hydrophila* into the peritoneal cavity. Dead fish were counted daily, and the challenge was allowed to continue until no mortality had occurred in any group for two days. Relative %-survival (RPS) was calculated using the formula:

RPS = [1-(%of dead fish in vaccinated group/% of dead fish in control group)]*100

When RPS≥60, the fish were considered protected by the vaccine.

+=protection, -=no protection

| | Challenge strains | Vaccine strains | | | | |
|---|---|---|---|---|---|---|
| | | AL20136 Biotype A | AL20212 Biotype B | AL20215 Biotype B | AL20136 Biotype A AL20212 Biotype B | AL20212 Biotype B AL20215 Biotype B |
| Challenge strains *A. hydrophila* | Biotype A AL 20136 | + | - | - | + | - |
| | Biotype A AL 20255 | + | - | - | + | - |
| | Biotype A AL 20258 | + | - | - | + | - |
| | Biotype A AL 20253 | + | - | - | + | - |
| | Biotype A AL 20250 | + | - | - | + | - |
| | Biotype A AL 20262 | + | - | - | + | - |
| | Biotype A AL 20257 | + | - | - | + | - |
| | Biotype A AL 20258 | + | - | - | + | - |
| | Biotype B AL 20215 | - | + | + | + | + |
| | Biotype B AL 20212 | - | + | + | + | + |

**Table 7**: Pangasius immunized with vaccines containing antigen from either biotype A, B or both were challenged with different biotype A or B strains. "+" indicates that the fish were protected by the vaccine. "-" indicates that the fish were not protected by the vaccine.

The results show that vaccines with antigen from biotype A protects against challenge with *Aeromonas hydrophila* belonging to biotype A, but not against challenge with *Aeromonas hydrophila* isolates belonging to biotype B.

Vaccines with antigen from biotype B protects against challenge with *Aeromonas hydrophila* belonging to biotype B, but not against challenge with *Aeromonas hydrophila* isolates belonging to biotype A.

If the vaccine contains antigen from both biotypes of *Aeromonas hydrophila,* the vaccine protects against challenge with *Aeromonas hydrophila* isolates from both biotype A and B.

**Example 6.** Combination vaccine against *Aeromonas hydrophila*

Vaccines (water in oil emulsions) with isolates of *Aeromonas hydrophila* belonging to biotype A or B and *Edwardsiella ictaluri* were made according to standard methods and pangasius were immunized by intraperiotoneal injection of 0,05ml. The concentration of antigen of *Aeromonas hydrophila* was $\geq 10^7$ bacteria/ml and the concentration of antigen of *E. ictaluri* was $\geq 10^9$ bacteria/ml. The pangasius were held under standard conditions in fresh water at 28 °C $\pm$ 2°C. 2 and 17 weeks after vaccination the fish were challenged by injecting 1000-6000 cfu of *Aeromonas hydrophila* biotype A or biotype B or $\geq 10^4$ *E. ictaluri* into the peritoneal cavity. Strains of *Aeromonas hydrophila* biotype A and B and *E. ictaluri* were used for challenge. Dead fish were counted daily, and the challenge was allowed to continue until no mortality had occurred in any group for two days.

Relative %-survival (RPS) was calculated using the formula

$$RPS = [1-(\% \text{ of dead fish in vaccinated group}/\% \text{ of dead fish in control group})]*100$$

When RPS$\geq$60, the fish were considered protected by the vaccine.

The results show that vaccines with combined antigens from *Aeromonas hydrophila* biotype A and B as well as *Edwardsiella ictaluri* protects against challenge with *Aeromonas hydrophila* belonging to biotype A and biotype B and *Edwardsialla ictaluri.*

**Table 8:** Fish were vaccinated with a vaccine containing antigens from *Aeromonas hydrophila* biotype A and B as well as *Edwardsiella ictaluri.* Protective effect of the vaccine was measured 2 and 17 weeks post vaccination by challenge with either *Aeromonas hydrophila* biotype A, *Aeromonas hydrophila* biotype B or *Edwardsiella ictaluri.* Vaccines giving relative percentage survival (RPS)$\geq$60 were considered protective. (+= protective vaccine, relative percentage survival (RPS)$\geq$60).

| | Challenge strains | | | | | |
| --- | --- | --- | --- | --- | --- | --- |
| | *A. hydrophila* biotype A | | *A. hydrophila* biotype B | | *E. ictaluri* | |
| Time of challenge (weeks post vaccination, wpv) | 2 wpv | 17 wpv | 2 wpv | 17 wpv | 2 wpv | 17 wpv |
| **Vaccine antigens** *A. hydrophila* biotype A, *A. hydrophila* biotype B *E. ictaluri* | + | + | Nd | + | + | + |

**Example 7:** . *Aeromonas hydrophila* vaccination and challenge in tilapia *(Oreochromis niloticus).*

The efficacy of a vaccine vaccine containing *Aeromonas hydrophila* biotype A, *Aeromonas hydrophila* biotype B and *Edwardsiella ictaluri* in tilapia *(Oreochromis niloticus)* was tested in a controlled laboratory trial where vaccinated and unvaccinated (control) fish were subjected to experimental challenge 120 tilapia were vaccinated with 0,05ml of a multivalent (water-in-oil emulsion) vaccine containing *Aeromonas hydrophila* biotype A, *Aeromonas hydrophila* biotype B and *Edwardsiella ictaluri.* The concentration of antigen was $\geq 10^7$ bacteria/ml. Vaccinated and control fish (120 tilapia) were marked and kept together in the same tank at 28°C.

3 weeks post vaccination, 30 fish from the vaccinated group and 30 fish from the unvaccinated control group were transferred to challenge tanks and were challenged with *A.hydrophila* biotype A in replicate tanks using two different challenge doses (tank 1-4). Dead fish were counted daily, and the challenge was allowed to continue until no mortality had occurred in any group for two days. Relative %-survival (RPS) was calculated using the formula:

$$RPS = [1-(\%\text{of dead fish in vaccinated group}/\%\text{ of dead fish in control group})]*100$$

| Challenge method | Injection | | | | | | | |
|---|---|---|---|---|---|---|---|---|
| Challenge dose | 7.4x10e5 cfu/fish | | | | 1.48x10e6 cfu/fish | | | |
| Tanks ID | Tank 1 | | Tank 2 | | Tank 3 | | Tank 4 | |
| Group | Vaccine | Control | Vaccine | Control | Vaccine | Control | Vaccine | Control |
| Mark | UM | PVR | UM | PVR | UM | PVR | UM | PVR |
| Challenge Strain | A.h F2O3(AL 20136) | | | | | | | |
| Total Fish dead | 1 | 12 | 1 | 14 | 1 | 22 | 0 | 21 |
| Total fish alive | 29 | 18 | 29 | 16 | 29 | 8 | 30 | 9 |
| Total no of fish | 30 | 30 | 30 | 30 | 30 | 30 | 30 | 30 |
| % Total mortality | 3.33 | 40 | 3.33 | 46.67 | 3.33 | 73.3 | 0 | 70 |
| RPS end | 91.67 | | 92.85 | | 95.45 | | 100 | |

Conclusion: *Aeromonas hydrophila* biotype A was virulent in tilapia. A multivalent vaccine containing *Aeromonas hydrophila* biotype A elicited protection against challenge with *Aeromonas hydrophila* biotype A.

**Example 8.** *Aeromonas hydrophila* vaccination and challenge in different fish species.

Vaccines containing *Aeromonas hydrophila* antigen will be made, and vaccination of fish performed by intraperitoneal injection. The fish species to be used are carps represented by common carp (*Cyprinus carpio*) and rohu (*Labeo rohita*), tilapias represented by *Oreochromis niloticus* and barramundi (*Latex calcarifer*). 2-4 weeks after vaccination, the fish are challenged with at least one virulent strain of *Aeromonas hydrophila,* and mortality in an unvaccinated control group is compared to mortality in the vaccinated fish. Relative percentage survival is calculated, and vaccines with RPS $\geq$ 60 are considered efficient.

**Receipt for deposits of microorganisms**

The following strains have been deposited under the "Budapest Treaty on the international Recognition of the Deposit of Microorganisms for the Purposes of Patent Procedure of 28 April 1977".

On 29 August 2012 the following bacterial strains were deposited by the inventors at the depositary institution "Health Protection Agency Culture Collections, Porton Down, Salisbury, Wiltshire, SP4 0JG, UK" under the following accession numbers:

*Aeromonas hydrophila,* biotype A (AL 20133) deposited under accession number 12082901
*Aeromonas hydrophila,* biotype B (AL 20215) deposited under accession number 12082902

**References**

(i) Martino ME, Fasolato L, Montemurro F, Rosteghin M, Manfrin A, Patarnello T, Novelli E, Cardazzo B. Appl Environ Microbiol. 2011 Jul;77(14):4986-5000. Epub 2011 Jun 3.
(ii) Janda JM, Abbott SL. Clin Microbiol Rev. 2010 Jan;23(1):35-73. Review.
(iii) http://pubmlst.org/aeromonas,
(iv) Tamura K, Peterson D, Peterson N, Stecher G, Nei M, Kumar S. Mol Biol Evol. 2011 Oct;28(10):2731-9. Epub 2011 May 4.
(v) Jan Raa (1996), Reviews in Fisheries Science 4(3): 229-228.
(vi) JC Aguilar and EG Rodriguez, 2007, Vaccine 25, 3752 - 3762

(vii) Santos Y, Toranzo AE, Barja JL, Nieto TP, Villa TG. Infect Immun. 1988 Dec;56(12):3285-93.
Pridgeon JW, Klesius PH. Dis Aquat Organ. 2011 Jul 12;95(3):209-15
(viii) Esteve C, Amaro C, Toranzo AE. FEMS Microbiol Lett. 1994 Mar 15;117(1):85-90
(ix) Zheng W, Cao H and Yang X (2012) African Journal of Microbiology Research, Vol 6 (21) pp 4512-4520
(x) Pridgeon JW, Klesius PH, Mu X, Carter D, Fleming K, Xu D, Srivastava K, Reddy G (2011) Veterinary microbiology 152 (117-125)
(xi) Chandran M. R. et al (2002) Fish and Shell Fish Immunology 13(1): p1-9
(Xii) Crumlish M. et al. (2010) J. Fish Dis. 33(9): p717-722
(xiii) Pridgeon J. W. et al (Vaccine 2011) Vaccine 29(45): p7896-7984

**Sequence listing**

**SEQ ID NO. 1 : DNA sequences of the genes coding for gltA in biotype A (AL20133)**

>20133_gltA

TTCCTGCACATGATGTTTGGCGTGCCGACCGAAGAGTACA

AGGTCAACCCCATCGTCGAACGCGCCATGGACCGCATCTT

CACCCTGCATGCGGATCACGAGCAGAACGCCTCCACCTCC

ACCGTGCGTCTGGCCGGCTCGTCCGGTGCCAACCCGTTCG

CCTGTATCGCCGCGGGGATCGCCTCCCTGTGGGGACCGGC

CCACGGCGGTGCCAACGAAGCCTGCCTCAAGATGCTCGAG

GAGATCGGCTCCGTGGATCGCATCCCCGAGTACATCGCCA

AGGCCAAGGACAAGAACGATCCGTTCCGTCTGATGGGCTT

CGGTCACCGGGTCTACAAGAACCACGACCCCCGTGCCACC

GTGATGCGCGAAACCTGTCACGAGGTACTGACCGAGCTGC

AGATCAAGGATCCGCTGCTGGACGTGGCCATGGAGCTGGA

GCGCATCGCGCTGTCCGACCCCTACTTCATCGAGAAGAAG

CTCTACCCGAACGTG

**SEQ ID NO. 2: DNA sequences of the genes coding for metG in biotype** A **(AL20133)**

>20133_metG

GCCGATATCTGGGTTCGTTATCAGCGAATGCGCGGTCACC

AGGTGCACTTTGTCTGTGCCGACGATGCCCATGGCACCCC

GATCATGCTCAAGGCCCAACAGCTGGGGATCACCCCGGAA

GAGATGATCGCGGCAGTCTCCCAGGAGCACCAGGCCGACT

TCGCCGGGTTCAACATCAGTTTTGACAACTATCACTCCAC

CCACAGCGACGAGAACCGTGAGCTGGCCGGGCTTATCTAC

GGCCGTCTGCAGGCCGGCGGCAAGATCAAAAGCCGCACCA

TCTCCCAGCTGTTCGATCCGGAAAAATCCATGTTCCTGCC

GGATCGTTTCGTCAAGGGCACCTGCCCCAAGTGCAAGTCC

CCGGAGCAGTATGGCGACAACTGCGACAGCTGCGGCGCTA

CCTACAGCCCGACCGAACTGATCGATCCGAAATCCGCCGT

CTCCGGCGCTACCCCGGTGATGAAGGACTCCGAGCACTTC

TTCTTCGACCTGCCGCAGTTTGAG

**SEQ ID NO. 3: DNA sequences of the genes coding for gltA in biotype B (AL20215)**

>20215_gltA

TTCCTGCACATGATGTTTGGCGTGCCGACCGAGGAGTACA

AGGTCAACCCCATCGTCGAACGCGCCATGGACCGCATCTT

TACCCTGCATGCGGACCACGAGCAGAACGCCTCCACCTCC

ACCGTGCGTCTGGCCGGCTCCTCCGGCGCCAACCCGTTTG

CCTGTATCGCCGCGGGGATCGCCTCCCTGTGGGGACCGGC

CCACGGTGGTGCCAACGAAGCCTGCCTCAAGATGCTCGAG

GAGATCGGCTCCGTTGACCGCATTCCCGAATACATCGCCA

AGGCCAAGGACAAGAACGATCCATTCCGTCTGATGGGCTT

CGGTCACCGGGTCTACAAGAACCACGACCCCCGTGCCACC

GTGATGCGCGAAACCTGTCACGAGGTACTGACCGAGCTGC

AGATCAAGGATCCGCTGCTGGACGTGGCCATGGAGCTGGA

GCGCATCGCGCTCTCCGACCCCTACTTCATCGAGAAGAAG

CTCTACCCGAACGTG

**SEQ ID NO. 4: DNA sequences of the genes coding for metG in biotype B (AL20215)**

>20215_metG

GCCGATATCTGGGTTCGTTATCAGCGAATGCGCGGTCACC

AGGTGCACTTTGTCTGTGCCGACGATGCCCACGGCACCCC

GATAATGCTCAAGGCCCAACAGCTGGGGATCACCCCGGAA

GAGATGATCGCGGCCGTCTCCAAGGAACACCAGGCCGACT

TCGCCGGGTTCAACATCAGTTTTGACAACTATCACTCCAC

CCACAGCGACGAGAACCGCGAGCTGGCGGAGCTGATCTAC

GGCCGTCTGCAGGCCGGCGGCAAGATCAAGAGCCGCACCA

TCTCCCAGCTGTTCGATCCGGAAAAGTCCATGTTCCTGCC

GGATCGCTTCGTCAAGGGTACCTGCCCCAAGTGCAAGTCC

CCGGAGCAGTATGGCGACAACTGTGACAGCTGCGGCGCCA

CCTACAGCCCGACCGAGCTGATTGATCCGAAATCCGCCGT

CTCCGGCGCCACCCCGGTGATGAAGGACTCCGAGCACTTC

TTCTTCGATCTGCCGCAGTTTGAA

**Claims**

1.  A composition comprising:

    (i) an immunogenic amount of a live attenuated or inactivated *Aeromonas hydrophila* biotype A bacterium; and
    (ii) an immunogenic amount of a live attenuated or inactivated *Aeromonas hydrophila* biotype B bacterium,

    wherein an *Aeromonas hydrophila* biotype A bacterium has a Methionyl-tRNA synthetase (metG) gene comprising a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1; and, wherein an *Aeromonas hydrophila* biotype B bacterium has a Methionyl-tRNA synthetase (metG) gene comprising a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

2.  A composition as claimed in claim 1, wherein:

    (a) an *Aeromonas hydrophila* biotype A bacterium comprises: a Methionyl-tRNA synthetase (metG) gene that comprises a sequence which is at least 99.5% identical to the sequence set forth in SEQ ID NO: 2; and/or a Citrate synthase I (gltA) gene, that comprises a sequence which is at least 99.5% identical to the sequence set forth in SEQ ID NO: 1;
    and/or,
    (b) an *Aeromonas hydrophila* biotype B bacterium comprises: a Methionyl-tRNA synthetase (metG) gene that comprises a sequence which is at least 99.5% identical to the sequence set forth in SEQ ID NO: 4; and/or a Citrate synthase I (gltA) gene, that comprises a sequence which is at least 99.5% identical to the sequence set forth in SEQ ID NO: 3.

3.  The composition according to either of claims 1 or 2, wherein:

    (a) an *Aeromonas hydrophila* biotype A bacterium is represented by the bacterium deposited under the "Budapest Treaty on the international Recognition of the Deposit of Microorganisms for the Purposes of Patent Procedure of 28 April 1977" at the Health Protection Agency Culture Collections, Porton Down, Salisbury, Wiltshire, SP4 0JG, UK under accession number 12082901; and/or,
    (b) an *Aeromonas hydrophila* biotype B bacterium is represented by the bacterium deposited under the "Budapest Treaty on the international Recognition of the Deposit of Microorganisms for the Purposes of Patent Procedure of 28 April 1977" at the Health Protection Agency Culture Collections, Porton Down, Salisbury, Wiltshire, SP4 0JG, UK under accession number 12082902.

4.  The composition according to any one of claims 1-3, wherein:

    (a) an *Aeromonas hydrophila* biotype A bacterium has the ability to induce clinical signs of septicaemia, and optionally, mortality in pangasius *(Pangasianodon hypophthalmus)* when injected intraperitoneally in fish weighing 10-20 grams in an amount of 100-2000 cfu/fish and an injection volume of 0.1 ml; and/or,
    (b) an *Aeromonas hydrophila* biotype B bacterium has the ability to induce clinical signs of septicaemia, and optionally, mortality in pangasius (*Pangasianodon hypophthalmus*) when injected intraperitoneally in fish weighing 10-20 grams in an amount of 100-6000 cfu/fish and an injection volume of 0.1 ml.

5.  The composition according to claim 4, wherein:

    (a) an *Aeromonas hydrophila* biotype A bacterium has the ability to induce at least 50% mortality in pangasius (*Pangasianodon hypophthalmus*) within 2-5 days after intraperitoneal injection of said bacterium in fish weighing 10-20 grams in an amount of 100-2000 cfu/fish and an injection volume of 0.1 ml; and/or,
    (b) an *Aeromonas hydrophila* biotype B bacterium has the ability to induce at least 50% mortality in pangasius (*Pangasianodon hypophthalmus*) within 2-5 days after intraperitoneal injection of said bacteria in fish weighing 10-20 grams in an amount of 100- 6000 cfu/fish and an injection volume of 0.1 ml.

6.  The composition according to any of claims 1 to 5, wherein the *Aeromonas hydrophila* biotype A and biotype B bacteria are:

(a) in a live attenuated form; or,
(b) in an inactivated form.

7. The composition according to any of claims 1 to 6, further comprising one or more additional antigens, optionally derived from the bacteria *Edwardsiella ictaluri, Edwardsiella tarda, Flavobacterium columnaris, Streptococcus iniae, Streptocossus agalactiae, Streptococcus dysgalactiae, Streptococcus parauberis.*

8. The composition according to any of claims 1 to 7, comprising amounts of $\geq 10^7$ bacteria/ml of *Aeromonas hydrophila* bacteria.

9. The composition according to claim 2, wherein:

(a) an *Aeromonas hydrophila* biotype A bacterium comprises: a Methionyl-tRNA synthetase (metG) gene that comprises a sequence which is 100% identical to the sequence set forth in SEQ ID NO: 2; and/or a Citrate synthase I (gltA) gene, that comprises a sequence which is 100% identical to the sequence set forth in SEQ ID NO: 1;
and/or,
(b) an *Aeromonas hydrophila* biotype B bacterium comprises: a Methionyl-tRNA synthetase (metG) gene that comprises a sequence which is 100% identical to the sequence set forth in SEQ ID NO: 4; and/or a Citrate synthase I (gltA) gene, that comprises a sequence which is 100% identical to the sequence set forth in SEQ ID NO: 3.

10. The composition according to any of claims 1 to 9, wherein the composition is in the form of a vaccine, and is optionally in a dosage form suitable for intraperitoneal injection, preferably wherein:

(a) the dosage form has a volume of between 0.01 to 0.2 ml/dosage; or,
(b) each dose comprises *Aeromonas hydrophila* bacterium in amounts of $\geq 10^7$ bacteria/ml.

11. A composition according to any of claims 1 to 10 for use in preventing or reducing the incidence of septicemia in fish and/or for use in preventing or reducing the incidence of *Aeromonas hydrophila* infections in fish, optionally wherein the fish is of the order Siluriformes, Perciformes and Cypriniformes, and wherein preferably:

(a) the fish is tilapia; or,
(b) the fish is pangasius (*Pangasianodon hypophthalmus*).

12. A composition comprising a bacterium of *Aeromonas hydrophila* biotype A, for use in preventing or reducing the incidence of septicemia in fish and/or for use in preventing or reducing the incidence of *Aeromonas hydrophila* infections in fish, wherein said composition is co-administered with a composition comprising a bacterium of *Aeromonas hydrophila* biotype B,
wherein an *Aeromonas hydrophila* biotype A bacterium has a Methionyl-tRNA synthetase (metG) gene comprising a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1; and, wherein an *Aeromonas hydrophila* biotype B bacterium has a Methionyl-tRNA synthetase (metG) gene comprising a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

13. A composition comprising a bacterium of *Aeromonas hydrophila* biotype B, for use in preventing or reducing the incidence of septicemia in fish and/or for use in preventing or reducing the incidence of *Aeromonas hydrophila* infections in fish, wherein said composition is co-administered with a composition comprising a bacterium of *Aeromonas hydrophila* biotype A,
wherein an *Aeromonas hydrophila* biotype A bacterium has a Methionyl-tRNA synthetase (metG) gene comprising a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1; and, wherein an *Aeromonas hydrophila* biotype B bacterium has a Methionyl-tRNA synthetase (metG) gene comprising a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

14. A method of manufacturing a composition as defined in any one of claims 1-11, comprising combining:

(i) an immunogenic amount of a live attenuated or inactivated *Aeromonas hydrophila* biotype A bacterium; and

(ii) an immunogenic amount of a live attenuated or inactivated *Aeromonas hydrophila* biotype B bacterium,

wherein an *Aeromonas hydrophila* biotype A bacterium has a Methionyl-tRNA synthetase (metG) gene comprising a sequence which is at least 99% identical to the sequence set forth in SEQ ID NO: 2, and/or a Citrate synthase I (gltA) gene, that comprises a sequence which is at least 99% identical to the sequence set forth in SEQ ID NO: 1; and, wherein an *Aeromonas hydrophila* biotype B bacterium has a Methionyl-tRNA synthetase (metG) gene comprising a sequence which is at least 99% identical to the sequence set forth in SEQ ID NO: 4, and/or a Citrate synthase I (gltA) gene, that comprises a sequence which is at least 99% identical to the sequence set forth in SEQ ID NO: 3.

**Patentansprüche**

1. Zusammensetzung, umfassend:

(i) eine immunogene Menge eines lebenden attenuierten oder inaktivierten *Aeromonas-hydrophila-Biotyp-A*-Bakteriums
und
(ii) eine immunogene Menge eines lebenden attenuierten oder inaktivierten *Aeromonas-hydrophila*-Biotyp-B-Bakteriums,

wobei ein *Aeromonas-hydrophila*-Biotyp-A-Bakterium ein Methionyl-tRNA-Synthetase (metG)-Gen, welches eine Sequenz, die zu wenigstens 99% identisch zu der in SEQ ID NO:2 angegebenen Sequenz ist, umfasst, und/oder ein Citratsynthase-I (gltA)-Gen, welches eine Sequenz, die zu wenigstens 99% identisch zu der in SEQ ID NO:1 angegebenen Sequenz ist, umfasst, hat, und wobei ein *Aeromonas-hydrophila*-Biotyp-B-Bakterium ein Methionin-tRNA-Synthetase (metG)-Gen, welches eine Sequenz, die zu wenigstens 99% identisch zu der in SEQ ID NO:4 angegebenen Sequenz ist, umfasst, und/oder ein Citratsynthase-I (gltA)-Gen, welches eine Sequenz, die zu wenigstens 99% identisch zu der in SEQ ID NO:3 angegebenen Sequenz ist, umfasst, hat.

2. Zusammensetzung gemäß Anspruch 1, wobei:

(a) ein *Aeromonas-hydrophila*-Biotyp-A-Bakterium umfasst: ein Methionyl-tRNA-Synthetase (metG)-Gen, welches eine Sequenz, die zu wenigstens 99,5% identisch zu der in SEQ ID NO:2 angegebenen Sequenz ist, umfasst, und/oder ein Citratsynthase-I (gltA)-Gen, welches eine Sequenz, die zu wenigstens 99,5% identisch zu der in SEQ ID NO:1 angegebenen Sequenz ist, umfasst;
und/oder
(b) ein *Aeromonas-hydrophila*-Biotyp-B-Bakterium umfasst: ein Methionyl-tRNA-Synthetase (metG)-Gen, welches eine Sequenz, die zu wenigstens 99,5% identisch mit der in SEQ ID NO:4 angegebenen Sequenz ist, umfasst, und/oder ein Citratsynthase-I (gltA)-Gen, welches eine Sequenz, die zu wenigstens 99,5% identisch mit der in SEQ ID NO:3 angegebenen Sequenz ist, umfasst.

3. Zusammensetzung gemäß Anspruch 1 oder 2, wobei:

(a) ein *Aeromonas-hydrophila*-Biotyp-A-Bakterium durch das Bakterium repräsentiert wird, das gemäß dem "Budapester Vertrag über die internationale Anerkennung der Hinterlegung von Mikroorganismen für die Zwecke von Patentverfahren vom 28. April 1977" bei der the Health Protection Agency Culture Collections, Porton Down, Salisbury, Wiltshire, SP4 0JG, GB unter der Zugangsnummer 12082901 hinterlegt wurde; und/oder
(b) ein *Aeromonas-hydrophila*-Biotyp-B-Bakterium durch das Bakterium repräsentiert wird, das gemäß dem "Budapester Vertrag über die internationale Anerkennung der Hinterlegung von Mikroorganismen für die Zwecke von Patentverfahren vom 28. April 1977" bei der the Health Protection Agency Culture Collections, Porton Down, Salisbury, Wiltshire, SP4 0JG, GB unter der Zugangsnummer 12082902 hinterlegt wurde.

4. Zusammensetzung gemäß einem der Ansprüche 1-3, wobei:

(a) ein *Aeromonas-hydrophila*-Biotyp-A-Bakterium die Fähigkeit hat, klinische Anzeichen von Septikämie und gegebenenfalls Mortalität bei Pangasius (*Pangasianodon hypophthalamus*), wenn es intraperitoneal in Fisch mit einem Gewicht von 10-20 Gramm in einer Menge von 100-2000 KbE/Fisch und mit einem Injektionsvolumen von 0,1 ml injiziert wird, zu induzieren, und/oder

(b) ein *Aeromonas-hydrophila*-Biotyp-B-Bakterium die Fähigkeit hat, klinische Anzeichen von Septikämie und gegebenenfalls Mortalität bei Pangasius (*Pangasianodon hypophthalamus*), wenn es intraperitoneal in Fisch mit einem Gewicht von 10-20 Gramm in einer Menge von 100-6000 KbE/Fisch und mit einem Injektionsvolumen von 0,1 ml injiziert wird, zu induzieren.

5.  Zusammensetzung gemäß Anspruch 4, wobei:

    (a) *Aeromonas-hydrophila*-Biotyp-A-Bakterium die Fähigkeit hat, wenigstens 50% Mortalität bei Pangasius (*Pangasianodon hypophthalamus*) innerhalb von 2-5 Tagen nach intraperitonealer Injektion des Bakteriums in Fisch mit einem Gewicht von 10-20 Gramm in einer Menge von 100-2000 KbE/Fisch und mit einem Injektionsvolumen von 0,1 ml zu induzieren; und/oder

    (b) *Aeromonas-hydrophila*-Biotyp-B-Bakterium die Fähigkeit hat, wenigstens 50% Mortalität bei Pangasius (*Pangasianodon hypophthalamus*) innerhalb von 2-5 Tagen nach intraperitonealer Injektion der Bakterien in Fisch mit einem Gewicht von 10-20 Gramm in einer Menge von 100-6000 KbE/Fisch und mit einem Injektionsvolumen von 0,1 ml zu induzieren.

6.  Zusammensetzung gemäß einem der Ansprüche 1 bis 5, wobei die *Aeromonas-hydrophila-Biotyp-A-* und -Biotyp-B-Bakterien sind:

    (a) in einer lebenden attenuierten bzw. lebend attenuierten Form oder
    (b) in einer inaktivierten Form.

7.  Zusammensetzung gemäß einem der Ansprüche 1 bis 6, die außerdem ein oder mehrere zusätzliche Antigen/e umfasst, die gegebenenfalls von den Bakterien *Edwardsiella ictaluri, Edwardsiella tarda, Flavobacterium columnaris, Streptococcus iniae, Streptococcus agalactiae, Streptococcus dysgalactiae, Streptococcus parauberis* stammen.

8.  Zusammensetzung gemäß einem der Ansprüche 1 bis 7, die Mengen von $\geq 10^7$ Bakterien/ml *Aeromonas-hydrophila*-Bakterien umfasst.

9.  Zusammensetzung gemäß Anspruch 2, wobei:

    (a) ein *Aeromonas-hydrophila*-Biotyp-A-Bakterium umfasst: ein Methionyl-tRNA-Synthetase (metG)-Gen, welches eine Sequenz, die zu wenigstens 100% identisch zu der in SEQ ID NO:2 angegebenen Sequenz ist, umfasst, und/oder ein Citratsynthase-I (gltA)-Gen, welches eine Sequenz, die zu wenigstens 100% identisch zu der in SEQ ID NO:1 angegebenen Sequenz ist, umfasst; und/oder

    (b) ein *Aeromonas-hydrophila*-Biotyp-B-Bakterium umfasst: ein Methionyl-tRNA-Synthetase (metG)-Gen, welches eine Sequenz, die zu wenigstens 100% identisch zu der in SEQ ID NO:4 angegebenen Sequenz ist, umfasst, und/oder ein Citratsynthase-I (gltA)-Gen, welches eine Sequenz, die zu wenigstens 100% identisch zu der in SEQ ID NO:3 angegebenen Sequenz ist, umfasst.

10. Zusammensetzung gemäß einem der Ansprüche 1 bis 9, wobei die Zusammensetzung in der Form eines Impfstoffs ist und gegebenenfalls in einer Dosierungsform ist, die zur intraperitonealen Injektion geeignet ist, wobei vorzugsweise:

    (a) die Dosierungsform ein Volumen von zwischen 0,01 bis 0,2 ml/Dosierung hat oder
    (b) jede Dosis *Aeromonas-hydrophila*-Bakterium in Mengen von $\geq 10^7$ Bakterien/ml umfasst.

11. Zusammensetzung gemäß einem der Ansprüche 1 bis 10 zur Verwendung bei der Prävention oder Verringerung der Inzidenz von Septikämie bei Fisch und/oder zur Verwendung bei der Prävention oder Reduzierung der Inzidenz von *Aeromonas-hydrophila*-Infektionen bei Fischen, wobei der Fisch gegebenenfalls zu der Ordnung Siluriformes, Perciformes und Cypriniformes gehört und wobei vorzugsweise:

    (a) der Fisch Tilapia ist oder
    (b) der Fisch Pangasius (*Pangasianodon hypophthalmus*) ist.

**12.** Zusammensetzung, die ein Bakterium von *Aeromonas-hydrophila*-Biotyp A umfasst, zur Verwendung bei der Prävention oder Verringerung der Inzidenz von Septikämie bei Fischen und/oder zur Verwendung bei der Prävention oder Verringerung der Inzidenz von *Aeromonas-hydrophila*-Infektionen bei Fischen,
wobei die Zusammensetzung mit einer Zusammensetzung, die ein Bakterium von *Aeromonas-hydrophila*-Biotyp B umfasst, coverabreicht wird,
wobei ein *Aeromonas-hydrophila*-Biotyp-A-Bakterium ein Methionyl-tRNA-Synthetase (metG)-Gen, welches eine Sequenz umfasst, die zu wenigstens 99% identisch zu der in SEQ ID NO:2 angegebenen Sequenz ist, und/oder ein Citratsynthase-I (gltA)-Gen, welches eine Sequenz umfasst, die zu wenigstens 99% identisch zu der in SEQ ID NO:1 angegebenen Sequenz ist, hat, und
wobei ein *Aeromonas-hydrophila*-Biotyp-B-Bakterium ein Methionin-tRNA-Synthetase (metG)-Gen, welches eine Sequenz, die zu wenigstens 99% identisch zu der in SEQ ID NO:4 angegebenen Sequenz ist, umfasst, und/oder ein Citratsynthase-I (gltA)-Gen, welches eine Sequenz, die zu wenigstens 99% identisch zu der in SEQ ID NO:3 angegebenen Sequenz ist, umfasst, hat.

**13.** Zusammensetzung, die ein Bakterium von *Aeromonas-hydrophila*-Biotyp B umfasst, zur Verwendung bei der Prävention oder Verringerung der Inzidenz von Septikämie bei Fischen und/oder zur Verwendung bei der Prävention oder Verringerung der Inzidenz von *Aeromonas-hydrophila*-Infektionen bei Fischen,
wobei die Zusammensetzung mit einer Zusammensetzung, die ein Bakterium von *Aeromonas-hydrophila*-Biotyp A umfasst, coverabreicht wird,
wobei ein *Aeromonas-hydrophila*-Biotyp-A-Bakterium ein Methionyl-tRNA-Synthetase (metG)-Gen, welches eine Sequenz umfasst, die zu wenigstens 99% identisch zu der in SEQ ID NO:2 angegebenen Sequenz ist, und/oder ein Citratsynthase-I (gltA)-Gen, welches eine Sequenz umfasst, die zu wenigstens 99% identisch zu der in SEQ ID NO:1 angegebenen Sequenz ist, hat, und
wobei ein *Aeromonas-hydrophila*-Biotyp-B-Bakterium ein Methionin-tRNA-Synthetase (metG)-Gen, welches eine Sequenz, die zu wenigstens 99% identisch zu der in SEQ ID NO:4 angegebenen Sequenz ist, umfasst, und/oder ein Citratsynthase-I (gltA)-Gen, welches eine Sequenz, die zu wenigstens 99% identisch zu der in SEQ ID NO:3 angegebenen Sequenz ist, umfasst, hat.

**14.** Verfahren zur Herstellung einer Zusammensetzung, wie sie in einem der Ansprüche 1-11 definiert ist, umfassend Kombinieren:

(i) einer immunogenen Menge eines lebenden attenuierten oder inaktivierten *Aeromonas-hydrophila*-Biotyp-A-Bakteriums
und
(ii) einer immunogenen Menge eines lebenden attenuierten oder inaktivierten *Aeromonas-hydrophila*-Biotyp-B-Bakteriums,

wobei ein *Aeromonas-hydrophila*-Biotyp-A-Bakterium ein Methionyl-tRNA-Synthetase (metG)-Gen, welches eine Sequenz umfasst, die zu wenigstens 99% identisch zu der in SEQ ID NO:2 angegebenen Sequenz ist, und/oder ein Citratsynthase-I (gltA)-Gen, welches eine Sequenz umfasst, die zu wenigstens 99% identisch zu der in SEQ ID NO:1 angegebenen Sequenz ist, hat, und
wobei ein *Aeromonas-hydrophila*-Biotyp-B-Bakterium ein Methionin-tRNA-Synthetase (metG)-Gen, welches eine Sequenz, die zu wenigstens 99% identisch zu der in SEQ ID NO:4 angegebenen Sequenz ist, umfasst, und/oder ein Citratsynthase-I (gltA)-Gen, welches eine Sequenz, die zu wenigstens 99% identisch zu der in SEQ ID NO:3 angegebenen Sequenz ist, umfasst, hat.

**Revendications**

**1.** Composition comprenant :

(i) une quantité immunogène d'une bactérie *Aeromonas hydrophila* vivante de biotype A atténuée ou inactivée ; et
(ii) une quantité immunogène d'une bactérie *Aeromonas hydrophila* vivante de biotype B atténuée ou inactivée,

dans laquelle une bactérie *Aeromonas hydrophila* de biotype A présente un gène de méthionyl-ARNt synthétase (metG) comprenant une séquence qui est identique à au moins 99 % à la séquence représentée par SEQ ID NO : 2, et/ou un gène de citrate synthase I (gltA), qui comprend une séquence qui est identique à au moins 99 % à la séquence représentée par SEQ ID NO : 1 ; et,

dans laquelle une bactérie *Aeromonas hydrophila* de biotype B présente un gène de méthionyl-ARNt synthétase (metG) comprenant une séquence qui est identique à au moins 99 % à la séquence représentée par SEQ ID NO : 4, et/ou un gène de citrate synthase I (gltA), qui comprend une séquence qui est identique à au moins 99 % à la séquence représentée par SEQ ID NO : 3.

2. Composition selon la revendication 1, dans laquelle :

(a) une bactérie *Aeromonas hydrophila* de biotype A comprend : un gène de méthionyl-ARNt synthétase (metG) qui comprend une séquence qui est identique à au moins 99,5 % à la séquence représentée par SEQ ID NO : 2 ; et/ou un gène de citrate synthase I (gltA), qui comprend une séquence qui est identique à au moins 99,5 % à la séquence représentée par SEQ ID NO : 1 ;
et/ou
(b) une bactérie *Aeromonas hydrophila* de biotype B comprend : un gène de méthionyl-ARNt synthétase (metG) qui comprend une séquence qui est identique à au moins 99,5 % à la séquence représentée par SEQ ID NO : 4 ; et/ou un gène de citrate synthase I (gltA), qui comprend une séquence qui est identique à au moins 99,5 % à la séquence représentée par SEQ ID NO : 3.

3. Composition selon l'une ou l'autre des revendications 1 ou 2, dans laquelle :

(a) une bactérie *Aeromonas hydrophila* de biotype A est représentée par la bactérie déposée conformément au « traité de Budapest sur la reconnaissance internationale du dépôt des micro-organismes aux fins de la procédure en matière de brevets du 28 avril 1977 » au Health Protection Agency Culture Collections, Porton Down, Salisbury, Wiltshire, SP4 0JG, RU sous le numéro d'accès 12082901 ; et/ou
(b) une bactérie *Aeromonas hydrophila* de biotype B est représentée par la bactérie déposée conformément au « traité de Budapest sur la reconnaissance internationale du dépôt des micro-organismes aux fins de la procédure en matière de brevets du 28 avril 1977 » au Health Protection Agency Culture Collections, Porton Down, Salisbury, Wiltshire, SP4 0JG, RU sous le numéro d'accès 12082902.

4. Composition selon l'une quelconque des revendications 1 à 3, dans laquelle :

(a) une bactérie *Aeromonas hydrophila* de biotype A a la capacité d'induire des signes cliniques de septicémie et, optionnellement, une mortalité chez le pangasius (*pangasianodon hypophthalmus*) quand elle est injectée par voie intrapéritonéale dans un poisson pesant 10 à 20 grammes en une quantité de 100 à 2000 ufc/poisson et à un volume d'injection de 0,1 ml ; et/ou,
(b) une bactérie *Aeromonas hydrophila* de biotype B a la capacité d'induire des signes cliniques de septicémie et, optionnellement, une mortalité chez le pangasius (*pangasianodon hypophthalmus*) quand elle est injectée par voie intrapéritonéale dans un poisson pesant 10 à 20 grammes en une quantité de 100 à 6000 ufc/poisson et à un volume d'injection de 0,1 ml.

5. Composition selon la revendication 4, dans laquelle :

(a) une bactérie *Aeromonas hydrophila* de biotype A a la capacité d'induire une mortalité d'au moins 50 % chez le pangasius (*pangasianodon hypophthalmus*) en 2 à 5 jours après une injection intrapéritonéale de ladite bactérie dans un poisson pesant 10 à 20 grammes en une quantité de 100 à 2000 ufc/poisson et à un volume d'injection de 0,1 ml ; et/ou,
(b) une bactérie *Aeromonas hydrophila* de biotype B a la capacité d'induire une mortalité d'au moins 50 % chez le pangasius (*pangasianodon hypophthalmus*) en 2 à 5 jours après une injection intrapéritonéale de ladite bactérie dans un poisson pesant 10 à 20 grammes en une quantité de 100 à 6000 ufc/poisson et à un volume d'injection de 0,1 ml.

6. Composition selon l'une quelconque des revendications 1 à 5, dans laquelle les bactéries *Aeromonas hydrophila* de biotype A et de biotype B sont :

(a) sous une forme vivante atténuée ; ou,
(b) sous une forme inactivée.

7. Composition selon l'une quelconque des revendications 1 à 6, comprenant en outre un ou plusieurs antigènes supplémentaires, optionnellement dérivés des bactéries *Edwardsiella ictaluri, Edwardsiella tarda, Flavobacterium*

*columnaris, Streptococcus iniae, Streptocossus agalactiae, Streptococcus dysgalactiae, Streptococcus parauberis.*

**8.** Composition selon l'une quelconque des revendications 1 à 7, comprenant des quantités $\geq 10^7$ bactéries/ml de bactéries *Aeromonas hydrophila.*

**9.** Composition selon la revendication 2, dans laquelle :

(a) une bactérie *Aeromonas hydrophila* de biotype A comprend : un gène de méthionyl-ARNt synthétase (metG) qui comprend une séquence qui est identique à 100 % à la séquence représentée par SEQ ID NO : 2 ; et/ou un gène de citrate synthase I (gltA), qui comprend une séquence qui est identique à 100 % à la séquence représentée par SEQ ID NO : 1 ;
et/ou
(b) une bactérie *Aeromonas hydrophila* de biotype B comprend : un gène de méthionyl-ARNt synthétase (metG) qui comprend une séquence qui est identique à 100 % à la séquence représentée par SEQ ID NO : 4 ; et/ou un gène de citrate synthase I (gltA), qui comprend une séquence qui est identique à 100 % à la séquence représentée par SEQ ID NO : 3.

**10.** Composition selon l'une quelconque des revendications 1 à 9, dans laquelle la composition est sous la forme d'un vaccin, et est optionnellement sous une forme galénique appropriée pour une injection intrapéritonéale, de préférence dans laquelle :

(a) la forme galénique a un volume situé entre 0,01 et 0,2 ml/dose ; ou,
(b) chaque dose comprend une bactérie *Aeromonas hydrophila* en des quantités $\geq 10^7$ bactéries/ml.

**11.** Composition selon l'une quelconque des revendications 1 à 10 pour son utilisation dans la prévention ou la réduction de l'incidence d'une septicémie chez un poisson et/ou son utilisation dans la prévention ou la réduction de l'incidence des infections par *Aeromonas hydrophila* chez un poisson, optionnellement dans laquelle le poisson appartient à l'ordre des siluriformes, des perciformes et des cypriniformes, et dans laquelle de préférence :

(a) le poisson est le tilapia ; ou,
(b) le poisson est le pangasius (*pangasianodon hypophthalmus*).

**12.** Composition comprenant une bactérie *Aeromonas hydrophila* de biotype A, pour son utilisation dans la prévention ou la réduction de l'incidence d'une septicémie chez un poisson et/ou pour son utilisation dans la prévention ou la réduction de l'incidence des infections par *Aeromonas hydrophila* chez un poisson, dans laquelle ladite composition est co-administrée avec une composition comprenant une bactérie *Aeromonas hydrophila* de biotype B,
dans laquelle une bactérie *Aeromonas hydrophila* de biotype A présente un gène de méthionyl-ARNt synthétase (metG) comprenant une séquence qui est identique à au moins 99 % à la séquence représentée par SEQ ID NO : 2, et/ou un gène de citrate synthase I (gltA), qui comprend une séquence qui est identique à au moins 99 % à la séquence représentée par SEQ ID NO : 1 ; et,
dans laquelle une bactérie *Aeromonas hydrophila* de biotype B présente un gène de méthionyl-ARNt synthétase (metG) comprenant une séquence qui est identique à au moins 99 % à la séquence représentée par SEQ ID NO : 4, et/ou un gène de citrate synthase I (gltA), qui comprend une séquence qui est identique à au moins 99 % à la séquence représentée par SEQ ID NO : 3.

**13.** Composition comprenant une bactérie *Aeromonas hydrophila* de biotype B, pour son utilisation dans la prévention ou la réduction de l'incidence d'une septicémie chez un poisson et/ou pour son utilisation dans la prévention ou la réduction de l'incidence des infections par *Aeromonas hydrophila* chez un poisson, dans laquelle ladite composition est co-administrée avec une composition comprenant une bactérie *Aeromonas hydrophila* de biotype A,
dans laquelle une bactérie *Aeromonas hydrophila* de biotype A présente un gène de méthionyl-ARNt synthétase (metG) comprenant une séquence qui est identique à au moins 99 % à la séquence représentée par SEQ ID NO : 2, et/ou un gène de citrate synthase I (gltA), qui comprend une séquence qui est identique à au moins 99 % à la séquence représentée par SEQ ID NO : 1 ; et,
dans laquelle une bactérie *Aeromonas hydrophila* de biotype B présente un gène de méthionyl-ARNt synthétase (metG) comprenant une séquence qui est identique à au moins 99 % à la séquence représentée par SEQ ID NO : 4, et/ou un gène de citrate synthase I (gltA), qui comprend une séquence qui est identique à au moins 99 % à la séquence représentée par SEQ ID NO : 3.

**14.** Procédé de préparation d'une composition telle que définie dans l'une quelconque des revendications 1 à 11, comprenant la combinaison :

(i) d'une quantité immunogène d'une bactérie *Aeromonas hydrophila* vivante de biotype A atténuée ou inactivée ; et

(ii) d'une quantité immunogène d'une bactérie *Aeromonas hydrophila* vivante de biotype B atténuée ou inactivée,

dans laquelle une bactérie *Aeromonas hydrophila* de biotype A présente un gène de méthionyl-ARNt synthétase (metG) comprenant une séquence qui est identique à au moins 99 % à la séquence représentée par SEQ ID NO : 2, et/ou un gène de citrate synthase I (gltA), qui comprend une séquence qui est identique à au moins 99 % à la séquence représentée par SEQ ID NO : 1 ; et,

dans laquelle une bactérie *Aeromonas hydrophila* de biotype B présente un gène de méthionyl-ARNt synthétase (metG) comprenant une séquence qui est identique à au moins 99 % à la séquence représentée par SEQ ID NO : 4, et/ou un gène de citrate synthase I (gltA), qui comprend une séquence qui est identique à au moins 99 % à la séquence représentée par SEQ ID NO : 3.

Fig. 1

## comparative virulens of different serotype

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010099444 A
- US 2012258139 A, Crumlish M.

- CN 101642567

**Non-patent literature cited in the description**

- **PRIDGEON J. W. et al.** *Vaccine,* 2011
- **JC AGUILAR ; EG RODRIGUEZ.** *Vaccine,* 2007, vol. 25, 3752-3762
- **MARTINO ME ; FASOLATO L ; MONTEMURRO F ; ROSTEGHIN M ; MANFRIN A ; PATARNELLO T ; NOVELLI E ; CARDAZZO B.** *Appl Environ Microbiol.,* July 2011, vol. 77 (14), 4986-5000
- **JANDA JM ; ABBOTT SL.** *Clin Microbiol Rev.,* January 2010, vol. 23 (1), 35-73
- **TAMURA K ; PETERSON D ; PETERSON N ; STECHER G ; NEI M ; KUMAR S.** *Mol Biol Evol.,* October 2011, vol. 28 (10), 2731-9
- **JAN RAA.** *Reviews in Fisheries Science,* 1996, vol. 4 (3), 229-228
- **SANTOS Y ; TORANZO AE ; BARJA JL ; NIETO TP ; VILLA TG.** *Infect Immun.,* December 1988, vol. 56 (12), 3285-93
- **PRIDGEON JW ; KLESIUS PH.** *Dis Aquat Organ.,* 12 July 2011, vol. 95 (3), 209-15
- **ESTEVE C ; AMARO C ; TORANZO AE.** *FEMS Microbiol Lett.,* 15 March 1994, vol. 117 (1), 85-90
- **ZHENG W ; CAO H ; YANG X.** *African Journal of Microbiology Research,* 2012, vol. 6 (21), 4512-4520
- **PRIDGEON JW ; KLESIUS PH ; MU X ; CARTER D ; FLEMING K ; XU D ; SRIVASTAVA K ; REDDY G.** *Veterinary microbiology,* 2011, vol. 152, 117-125
- **CHANDRAN M. R. et al.** *Fish and Shell Fish Immunology,* 2002, vol. 13 (1), 1-9
- **CRUMLISH M. et al.** *J. Fish Dis.,* 2010, vol. 33 (9), 717-722
- **PRIDGEON J. W. et al.** *Vaccine 2011) Vaccine,* vol. 29 (45), 7896-7984